# EUROPÄISCHE PATENTSCHRIFT

(11) **EP 4 197 581 B1**
(45) Veröffentlichungstag und Bekanntmachung des Hinweises auf die Patenterteilung: **12.03.2025**
(21) Anmeldenummer: 22212763.1
(22) Anmeldetag: 12.12.2022
(51) Int. Cl.: A61M 16/12

(54) **ANORDNUNG ZUR VERSORGUNG EINER PATIENTENSEITIGEN KOPPELEINHEIT MIT EINEM GASGEMISCH**
ARRANGEMENT FOR SUPPLYING A PATIENT-SIDE COUPLING UNIT WITH A GAS MIXTURE
SYSTÈME POUR ALIMENTER UN ENSEMBLE DE COUPLAGE CÔTÉ PATIENT AVEC UN MÉLANGE GAZEUX

(30) Priorität: 14.12.2021 DE 102021132927
(43) Veröffentlichungstag der Anmeldung: 21.06.2023
(73) Patentinhaber: Drägerwerk AG & Co. KGaA, 23558 Lübeck (DE)
(72) Erfinder: Hansmann, Hans-Ullrich, 23558 Lübeck (DE); Garbrecht, Oliver, 23558 Lübeck (DE); Fischer, Hendrik, 23558 Lübeck (DE)

(56) Entgegenhaltungen:
- EP-B1- 1 795 222
- US-A- 3 664 361
- US-A- 3 762 427
- US-A- 4 549 563

## Beschreibung

Die Erfindung betrifft eine Versorgungs-Anordnung, um eine patientenseitige Koppeleinheit mit einem Gasgemisch zu versorgen.

Die Erfindung lässt sich beispielsweise für die künstliche Beatmung eines Patienten verwenden. Im oder am Körper des Patienten ist eine patientenseitige Koppeleinheit angeordnet, beispielsweise eine Atemmaske, ein Katheter oder ein Tubus. Um den Patienten künstlich zu beatmen, wird ein Gasgemisch zu der patientenseitigen Koppeleinheit gefördert. Dieses Gasgemisch umfasst Sauerstoff und in einer Ausgestaltung mindestens ein Narkosemittel. Bevorzugt führt ein Beatmungsgerät eine Abfolge von Beatmungshüben aus und fördert in jedem Beatmungshub eine Menge des Gasgemischs zur patientenseitigen Koppeleinheit.

Möglich ist, dass als Gasgemisch Atemluft verwendet wird. Oft wird gewünscht, dass der Anteil von Sauerstoff in demjenigen Gasgemisch, welches zur patientenseitigen Koppeleinheit gefördert wird, größer als der Sauerstoffanteil in der Atemluft ist und einem vorgegebenen zeitlichen Verlauf folgt, beispielsweise zeitlich konstant ist. Um dieses Ziel zu erreichen, wird ein Gasgemisch umfassend Atemluft und reinem Sauerstoff erzeugt. Die Erfindung lässt sich dafür anwenden, um ein solches Gasgemisch zu erzeugen.

Eine Versorgungs-Anordnung mit den Merkmalen des Oberbegriffs des Anspruchs 1 ist aus US 3 664 361 A bekannt. Dort wird eine Beatmungs-Anordnung (respiration system) beschrieben, die Sauerstoff von einer druckgeregelten Quelle (pressure regulated source 10) mit Luft von einem Lufteinlass (air intake 11) vermischt und das Ergebnis der Vermischung an einen Patienten leitet. Die Luft wird mittels einer Pumpe 13 vom Einlass 11 über eine erste Leitung 14, einem Bauteil (manifold 19) und einer zweiten Leitung 15 zu einer Mischeinheit (proportioning device 20) gefördert. Der Sauerstoff wird über Leitungen 41, 53, 32 zur Mischeinheit 20 gefördert. Ein mechanischer Regler (control apparatus 33) passt den Druck des Sauerstoffs in der Leitung 32 an abgetastete Schwankungen des Drucks in der Leitung 14 an. Der Regler 33 umfasst ein Reservoir 35 mit variablem Volumen, wobei das Reservoir 35 eine feste Wand 43, eine bewegliche Wand 45 und zwei faltbare Wände 47 umfasst und wobei im Reservoir 35 ein Überdruck gegenüber der Umgebung herrscht. Ein Ausgleichsventil (balancing valve 37) öffnet und schließt eine Ausgleichsöffnung (balancing port 38) in einer Wand 43 zwischen dem Reservoir 35 und der Leitung 32. Eine Membran (diaphragm 39) ist auf der einen Seite dem Druck in der Leitung 32 ausgesetzt und auf der anderen Seite einem Druck, der über eine Leitung 79 dem Luftdruck in der Leitung 14 ausgesetzt ist.

Die Beatmungs-Anordnung von US 4 549 563 A fördert ein Gasgemisch umfassend Sauerstoff aus einer Quelle 11 und Stickstoff aus einer Quelle 21 und optional ein Narkosemittel zu einer Atemmaske auf dem Gesicht eines Patienten. Die Bestandteile des Gasgemisch werden in einer Mischeinheit (circuit 3) miteinander vermischt. Ein Druckminderer (pressure limiter 40) umfasst zwei Kammern 42 und 43 sowie eine Membrane (diaphragm 41) zwischen den beiden Kammern 42 und 43. Die Kammer 42 ist mit der Quelle 21 und mit einer Leitung 20 verbunden, wobei die Leitung 20 zur Mischeinheit 3 führt. Die Kammer 43 ist über eine Leitung 46 mit einer Leitung 10 verbunden, wobei die Leitung 10 die Quelle 11 mit der Mischeinheit 3 verbindet.

Das Beatmungsgerät 14 von EP 2 425 869 A1 umfasst eine Mischvorrichtung und einen Ventilationsteil. In der Mischvorrichtung wird ein medizinisches Gas, beispielsweise Sauerstoff oder ein Narkosemittel, mit Luft vermischt. Das medizinische Gas wird über einen Gaseinlass B eingespeist und über eine Gasleitung 10 zugeführt. Die Luft wird über einen Lufteinlass A eingespeist und über eine Luftleitung 11 zugeführt. In der Gasleitung 10 sind ein Reduktionsventil 6, ein Sicherheitsventil 7, ein ansteuerbares Proportionalventil 8 und ein Durchflussmesser 9 angeordnet. In der Luftleitung 11 sind ein Gebläse 1 und ein Rückschlagventil 2 angeordnet. Der Ventilationsteil leitet das Gasgemisch über eine Atemgasleitung 12 einem Patienten als Atemgas zu. In der Atemgasleitung 12 sind ein Durchflusssensor 3, ein ansteuerbares Proportionalventil 4 und ein Drucksensor 5 angeordnet. Eine Steuereinheit 13 empfängt Signale von den Sensoren 9, 3 und vermag alle ansteuerbaren Ventile 8, 4 anzusteuern. Die Ansteuerung wird mit dem Regelungsziel durchgeführt, dass das Gasgemisch in der Atemgasleitung 12 eine gewünschte Zusammensetzung aus der Luft und dem medizinischen Gas aufweist.

Der Erfindung liegt die Aufgabe zugrunde, eine Versorgungs-Anordnung mit den Merkmalen des Oberbegriffs des Anspruchs 1 bereitzustellen, welche eine patientenseitige Koppeleinheit mit einem Gasgemisch umfassend zwei Gas-Bestandteile zu versorgen vermag, wobei der zeitliche Verlauf des Volumenflusses und / oder des Drucks des bereitgestellten Gasgemisches in einer Fluidverbindung zu der patientenseitigen Koppeleinheit sich relativ zuverlässig regeln lässt.

Die Aufgabe wird durch eine Versorgungs-Anordnung mit den Merkmalen des Anspruchs 1 gelöst. Vorteilhafte Ausgestaltungen sind in den Unteransprüchen angegeben.

Die erfindungsgemäße Versorgungs-Anordnung vermag eine patientenseitige Koppeleinheit mit einem Gasgemisch zu versorgen. Dieses Gasgemisch umfasst einen ersten Gas-Bestandteil, insbesondere Luft, und einen zweiten Gas-Bestandteil, insbesondere reinen Sauerstoff. Möglich ist auch, dass ein Gas-Bestandteil ein Narkosemittel ist. Der zweite Gas-Bestandteil unterscheidet sich chemisch vom ersten Gas-Bestandteil. Möglich ist, dass beide Gas-Bestandteile dieselbe Komponente umfassen, beispielsweise beide Sauerstoff umfassen.

Die patientenseitige Koppeleinheit ist wenigstens zeitweise mit einem Patienten verbunden oder lässt sich mit dem Patienten verbunden. Insbesondere ist die patientenseitige Koppeleinheit wenigstens zeitweise im oder am Körper des Patienten angeordnet oder lässt sich dort anordnen. Ein Tubus, ein Katheter und eine Atemmaske sind Beispiele für eine patientenseitige Koppeleinheit.

Nachfolgend wird der Begriff "Kanal" verwendet. Unter einem Kanal wird ein Bauteil verstanden, welches ein Fluid, insbesondere ein Gas oder Gasgemisch, entlang einer vorgegebenen Trajektorie zu führen vermag und idealerweise verhindert, dass das Fluid diese Trajektorie verlässt. Ein Schlauch und eine Röhre sind Beispiele für einen Kanal. In der Regel ist die beabsichtigte Nutzung eines Kanals, dass Fluid stets oder wenigstens zeitweise in ein und dieselbe Richtung durch den Kanal geleitet wird, aber nicht in die entgegengesetzte Richtung.

Außerdem ist nachfolgend davon die Rede, dass eine Fluidverbindung zwischen zwei Bauteilen hergestellt ist. Darunter wird verstanden, dass ein Fluid vom dem einen Bauteil zu dem anderen Bauteil fließen kann, idealerweise ohne in die Umgebung zu entweichen. Möglich ist, dass die beiden Bauteile direkt miteinander verbunden sind. Möglich ist auch, dass ein Abstand zwischen den beiden Bauteilen auftritt und eine Fluidführungseinheit, beispielsweise ein Schlauch und / oder ein Kanal im obigen Sinne, die beiden Bauteile miteinander verbindet. Möglich ist, dass ein Fluid zeitweise vom ersten Bauteil durch die Fluidverbindung hindurch zum zweiten Bauteil und zeitweise umgekehrt vom zweiten Bauteil durch die Fluidverbindung hindurch zum ersten Bauteil fließt. Die Fluidverbindung kann dauerhaft oder nur zeitweise hergestellt sein.

Weiterhin wird nachfolgend der Begriff "Quelle" verwendet. Im Kontext der Erfindung vermag eine Quelle dauerhaft oder wenigstens zeitweise ein Fluid bereitzustellen, insbesondere einen Gas-Bestandteil des Gasgemischs. Eine Quelle ist insbesondere ein stationärer Versorgungsanschluss oder eine mobile Quelle, beispielsweise ein Behälter mit dem Fluid, insbesondere eine Druckluftflasche. Möglich ist auch, dass eine Fluidfördereinheit, beispielsweise eine Pumpe oder ein Gebläse eines Beatmungsgeräts, als eine Quelle für ein Fluid fungiert. Möglich ist, dass zwei verschiedenartige Quellen für denselben Gas-Bestandteil verwendet werden, insbesondere eine stationäre Quelle und eine mobile Quelle.

Die erfindungsgemäße Versorgungs-Anordnung umfasst einen ersten Kanal mit einem Versorgungs-Verbindungselement, welches als Eingang zu dem ersten Kanal fungiert. Zwischen dem Versorgungs-Verbindungselement und einer ersten Quelle ist wenigstens zeitweise eine Fluidverbindung hergestellt oder lässt sich herstellen. Die erste Quelle vermag den ersten Gas-Bestandteil bereitzustellen. Der erste Kanal vermag den ersten Gas-Bestandteil vom Versorgungs-Verbindungselement zu einem Mischpunkt der Versorgungs-Anordnung zu leiten. Der Druck im ersten Kanal zu einem Zeitpunkt kann entlang des ersten Kanals variieren. In der Regel variiert der Druck an einem Punkt im ersten Kanal über der Zeit.

Weiterhin umfasst die Versorgungs-Anordnung einen Druckminderer mit einem Vordruck-Eingang und einem Hinterdruck-Ausgang. Unter einem Druckminderer wird ein Bauteil verstanden, welches an seinem Vordruck-Eingang ein Gas, vorliegend den zweiten Gas-Bestandteil, mit einem Vordruck empfängt und dieses Gas mit einem Hinterdruck an seinem Hinterdruck-Ausgang bereitstellt, wobei der Hinterdruck kleiner als oder gleich dem Vordruck ist und wobei der Hinterdruck stets kleiner oder gleich einer vorgegebenen Hinterdruck-Schranke ist, egal wie groß der Vordruck ist. Die Hinterdruck-Schranke kann durch die Konstruktion des Druckminderers vorgegeben sein. In der Regel ist der Hinterdruck zeitlich veränderlich, und auch der Vordruck kann zeitlich veränderlich sein. Wenigstens zeitweise leitet der Druckminderer das Gas vom Vordruck-Eingang durch sein Inneres hindurch zum Hinterdruck-Ausgang.

Zwischen dem Vordruck-Eingang und einer zweiten Quelle ist wenigstens zeitweise eine Fluidverbindung hergestellt oder lässt sich herstellen. Die zweite Quelle vermag den zweiten Gas-Bestandteil bereitzustellen. Dank der Fluidverbindung kann der zweite Gas-Bestandteil von der zweiten Quelle zum Vordruck-Eingang fließen.

Der Druckminderer vermag den zweiten Gas-Bestandteil an seinem Hinterdruck-Ausgang bereitzustellen. Der Druckminderer ist dazu ausgestaltet, den zweiten Gas-Bestandteil wie folgt bereitzustellen: Der zeitliche Verlauf des Drucks am Hinterdruck-Ausgang folgt dem zeitlichen Verlauf des Drucks an einem Referenzpunkt im ersten Kanal. Dieser Referenzpunkt kann im Versorgungs-Verbindungselement liegen, mit dem Mischpunkt übereinstimmen oder sich zwischen dem Versorgungs-Verbindungselement und dem Mischpunkt befinden.

Das Merkmal, dass der zeitliche Verlauf einer physikalischen Größe B dem zeitlichen Verlauf einer physikalischen Größe A folgt, bedeutet folgendes: Falls A zunimmt oder abnimmt, so nimmt B auch zu bzw. ab, in der Regel mit einer gewissen zeitlichen Verzögerung. Mindestens dann, wenn A für einen ausreichend langen Zeitraum den gleichen Wert beibehält, A also in diesem Zeitraum konstant bleibt, nimmt B wenigstens am Ende dieses Zeitraums ebenfalls diesen Wert an.

Die Versorgungs-Anordnung umfasst weiterhin einen zweiten Kanal. Der Hinterdruck-Ausgang des Druckminderers ist mit dem zweiten Kanal pneumatisch verbunden und / oder steht in einer Fluidverbindung mit dem zweiten Kanal. Der zweite Kanal vermag den zweiten Gas-Bestandteil vom Hinterdruck-Ausgang des Druckmischers zum Mischpunkt zu leiten.

Die Gas-Bestandteile, die von den beiden Kanälen zum Mischpunkt geleitet werden, werden im Mischpunkt zum Gasgemisch vermischt oder vermischen sich von alleine zum Gasgemisch.

Außerdem umfasst die Versorgungs-Anordnung einen Inspirations-Kanal, der vom Mischpunkt zur patientenseitigen Koppeleinheit führt. Der Inspirations-Kanal vermag ein Gasgemisch, das im Mischpunkt erzeugt ist oder wird oder im Mischpunkt von allein entsteht, zur patientenseitigen Koppeleinheit zu leiten.

Erfindungsgemäß münden also der erste Kanal und der zweite Kanal in den Mischpunkt, und der Inspirations-Kanal beginnt im Mischpunkt. Im einfachsten Fall ist der Mischpunkt ein rein mechanisches Bauteil, das diese drei Kanäle nach Art eines Y-Stücks miteinander verbindet. Möglich ist auch, dass der Mischpunkt durch einen Gasmischer realisiert wird, welcher die beiden Gas-Bestandteile zu einem idealerweise homogenen Gasgemisch vermischt.

Die Erfindung ermöglicht es, ein Gasgemisch umfassend mindestens zwei Gas-Bestandteile zur patientenseitigen Koppeleinheit zu fördern und dadurch an der patientenseitigen Koppeleinheit das Gasgemisch für die künstliche Beatmung eines Patienten bereitzustellen. Weil die Versorgungs-Anordnung dazu ausgestaltet ist, das Gasgemisch aus mindestens zwei Gas-Bestandteilen zusammenzusetzen, ermöglicht die Erfindung in vielen Fällen, ein Gasgemisch bereitzustellen, welches auf die aktuell erforderliche künstliche Beatmung des Patienten zugeschnitten ist. Insbesondere kann reiner Sauerstoff als der zweite Gas-Bestandteil fungieren, und der Anteil von Sauerstoff im Gasgemisch lässt sich einstellen und bei Bedarf verändern. Der zweite Gas-Bestandteil kann auch ein Narkosemittel sein, und der Anteil des Narkosemittels im Gasgemisch lässt sich einstellen und bei Bedarf verändern.

Weil zwischen der zweiten Quelle und dem zweiten Kanal ein Druckminderer angeordnet ist, wird ermöglicht, dass die zweite Quelle den zweiten Gas-Bestandteil mit einem höheren Druck bereitstellt als die erste Quelle den ersten Gas-Bestandteil. Weil der Druck am Hinterdruck-Ausgang und damit im zweiten Kanal dem Druck am Referenzpunkt im ersten Kanal folgt, wird in der Regel verhindert, dass am Mischpunkt ein großer Druckunterschied auftritt, auch wenn die beiden Quellen die beiden Gas-Bestandteile mit sehr unterschiedlichen Drücken bereitstellen. Der Druck, mit dem die zweite Quelle den zweiten Gas-Bestandteil bereitstellt, kann dank des Druckminderers auch höher sein als der maximal zulässige Druck des Gasgemischs an der patientenseitigen Koppeleinheit oder im ersten oder im zweiten Kanal.

Diese Wirkungen des Druckminderers sind insbesondere dann von Vorteil, wenn die erste Quelle eine Fluidförderereinheit eines medizinischen Geräts und die zweite Quelle ein stationärer Versorgungsanschluss ist oder eine unter Druck stehende Flasche mit dem zweiten Gas-Bestandteil umfasst. Beispiele für eine solche Fluidfördereinheit sind ein Gebläse oder eine Pumpe. Häufig liefert die erste Quelle den ersten Gas-Bestandteil mit einem Druck, der zwischen 20 mbar und 100 mbar liegt. Die zweite Quelle liefert den zweiten Gas-Bestandteil häufig mit einem Druck, der zwischen 2 bar und 8 bar liegt. Möglich, aber dank der Erfindung nicht erforderlich ist es, an der zweiten Quelle den Druck zu reduzieren oder zu steuern oder zu regeln.

Der Druckminderer stellt an seinem Hinterdruck-Ausgang den zweiten Gas-Bestandteil mit einem Druck bereit, der niedriger als oder höchstens genau so groß wie der Druck am Vordruck-Eingang ist. Der Druck, mit dem der zweite Gas-Bestandteil am Hinterdruck-Ausgang bereitgestellt wird, folgt erfindungsgemäß dem Druck des ersten Gas-Bestandteils am Referenzpunkt im ersten Kanal. Der Druck im ersten Kanal ist also der Master und der Druck im zweiten Kanal der Slave.

Dieses erfindungsgemäße Merkmal erleichtert es, den zeitlichen Verlauf des Drucks im Inspirations-Kanal oder des Volumenflusses durch den Inspirations-Kanal, also flussabwärts vom Mischpunkt, zu regeln (closed-loop control). Eine solche Regelung wird häufig mit dem Regelungsziel (control gain) durchgeführt, dass der tatsächliche zeitliche Verlauf des Drucks oder des Volumenflusses an der patientenseitigen Koppeleinheit einem vorgegebenen zeitlichen Sollverlauf folgt.

Außerdem erleichtert dieses Merkmal es in manchen Fällen, den zeitlichen Verlauf des Drucks oder des Volumenflusses flussaufwärts vom Mischpunkt, also im ersten Kanal und / oder im zweiten Kanal, zu regeln. Das Regelungsziel bei dieser Regelung ist in einer Ausgestaltung, dass der zeitliche Verlauf des tatsächlichen Drucks im oder des tatsächlichen Volumenflusses durch den jeweiligen Kanal einem vorgegebenen zeitlichen Sollverlauf folgt. In einer anderen Ausgestaltung ist das Regelungsziel, dass der Anteil mindestens eines Gas-Bestandteils im Gasgemisch in einem vorgegebenen Sollbereich liegt. In einer Realisierungsformen sollen beide Regelungsziele erreicht werden. In manchen Fällen reicht es aus, den Druck im ersten Kanal zu regeln. Der Druck im zweiten Kanal folgt dann dank der Erfindung dem geregelten Druck im ersten Kanal.

Dank der Erfindung erreichen die beiden Gas-Bestandteile mit idealerweise demselben zeitlichen Verlauf des Drucks den Mischpunkt. In der Praxis folgt der Druck im zweiten Kanal mit einem Zeitverzug dem Druck im ersten Kanal. Der Unterschied zwischen den beiden Drücken in den beiden Kanälen und damit der Druckunterschied am Mischpunkt bleibt dank der Erfindung in der Regel zu jedem Zeitpunkt relativ gering. Dank der Erfindung ist in vielen Fällen auch dann eine zuverlässige Regelung des Volumenflusses und / oder des Drucks und / oder des Mischungsverhältnisses im Gasgemisch möglich, wenn der Sollverlauf rasche Änderungen des Drucks und / oder des Volumenflusses vorgibt und / oder wenn der Druck, mit dem eine Quelle einen Gas-Bestandteil bereitstellt, zeitlich schwankt. In vielen Fällen ermöglicht die Erfindung eine Regelung, bei der eine Regelabweichung rasch reduziert wird. Außerdem reduziert die Erfindung die Gefahr, dass aufgrund eines starken Druckunterschieds zwischen den beiden Kanälen zum Mischpunkt in einem Kanal ein Rückstau auftritt, was häufig unerwünscht ist.

Um den zeitlichen Verlauf des Drucks im Inspirations-Kanal oder des Volumenflusses durch den Inspirations-Kanal zu regeln, reicht es in vielen Fällen aus, den zeitlichen Verlauf des Drucks bzw. des Volumenflusses im ersten Kanal zu regeln. Der zeitliche Verlauf des Drucks im bzw. des Volumenflusses durch den zweiten Kanal folgt dank der Erfindung dem zeitlichen Verlauf am Referenzpunkt im ersten Kanal, ohne dass auch für den zweiten Kanal eine Regelung erforderlich ist.

Dank des erfindungsgemäßen Druckminderers ist es nicht erforderlich, den Druck zu steuern oder zu regeln, mit dem die zweite Quelle den zweiten Gas-Bestandteil bereitstellt. Möglich ist vielmehr, dass die zweite Quelle den zweiten Gas-Bestandteil mit einem konstanten Druck oder einem Druck, der sich unabhängig vom Druck im ersten Kanal ändert, bereitstellt. Trotzdem lassen sich dank der Erfindung der Druck im und / oder der Volumenfluss durch den Inspirations-Kanal und / oder das Mischungsverhältnis im Gasgemisch in vielen Fällen relativ zuverlässig regeln. Der Druckminderer entkoppelt die zweite Quelle vom zweiten Kanal und damit auch vom ersten Kanal.

Erfindungsgemäß vermag ein ansteuerbares erstes Ventil den Volumenfluss durch den ersten Kanal oder den Druck im ersten Kanal zu verändern, beispielsweise mittels einer veränderlichen Querschnittsfläche (Proportionalventil). Ein signalverarbeitendes Steuergerät (control unit) vermag dieses erste Ventil anzusteuern. Dieses Steuergerät führt eine erste Regelung (closed-loop control) durch. Bei dieser ersten Regelung empfängt und verarbeitet das Steuergerät ein Signal eines ersten Sensors und steuert das erste Ventil abhängig von diesem Signal an.

In einer ersten Alternative dieser Ausgestaltung ist das Regelungsziel bei der ersten Regelung, dass der zeitliche Verlauf des tatsächlichen Drucks im ersten Kanal einem vorgegebenen Sollverlauf des Drucks folgt. Der erste Sensor vermag ein Maß für den Druck im ersten Kanal zu messen.

In einer zweiten Alternative dieser Ausgestaltung ist das Regelungsziel bei der ersten Regelung, dass der zeitliche Verlauf des tatsächlichen Volumenflusses durch den ersten Kanal einem vorgegebenen Sollverlauf des Volumenflusses folgt. Der erste Sensor vermag ein Maß für den Volumenfluss durch den ersten Kanal zu messen.

Diese beiden Alternativen lassen sich auch miteinander kombinieren. Der erste Sensor vermag dann sowohl ein Maß für den Druck als auch ein Maß für den Volumenfluss zu messen.

In einer Ausgestaltung führt das Steuergerät eine zweite Regelung durch, die den zweiten Kanal betrifft. Ein zweites Ventil vermag den Volumenfluss durch den und / oder den Druck im zweiten Kanal zu verändern. Das Steuergerät steuert das zweite Ventil abhängig von einem Signal eines zweiten Sensors an.

In einer ersten Alternative dieser Ausgestaltung ist das Regelungsziel bei der zweiten Regelung, dass der zeitliche Verlauf des tatsächlichen Drucks im zweiten Kanal einem vorgegebenen Sollverlauf des Drucks folgt. Der zweite Sensor vermag ein Maß für den Druck im zweiten Kanal zu messen.

In einer zweiten Alternative dieser Ausgestaltung ist das Regelungsziel bei der zweiten Regelung, dass der zeitliche Verlauf des tatsächlichen Volumenflusses durch den zweiten Kanal einem vorgegebenen Sollverlauf des Volumenflusses folgt. Der zweite Sensor vermag ein Maß für den Volumenfluss durch den zweiten Kanal zu messen.

Diese beiden Alternativen lassen sich auch miteinander kombinieren. Der zweite Sensor vermag dann sowohl ein Maß für den Druck als auch ein Maß für den Volumenfluss zu messen.

Erfindungsgemäß folgt der zeitliche Verlauf des Drucks am Hinterdruck-Ausgang des Druckminderers und damit im zweiten Kanal dem zeitlichen Verlauf des Drucks am Referenzpunkt im ersten Kanal. Dieses Merkmal erleichtert es in vielen Fällen, die beiden gerade beschriebenen Regelungen durchzuführen. Häufig lässt sich derselbe Regelalgorithmus für beide Regelungen verwenden.

Möglich ist auch, dass das Steuergerät den Volumenfluss durch den ersten und / oder den zweiten Kanal steuert.

Ein alternatives oder zusätzliches Regelungsziel bei einer dieser beiden Ausgestaltungen kann auch sein, dass der Anteil des ersten Gas-Bestandteils und / oder der Anteil des zweiten Gas-Bestandteils in dem Gasgemisch, welches im Mischpunkt entsteht oder erzeugt wird, einem vorgegebenen zeitlichen Verlauf erfolgt, insbesondere zeitlich konstant ist. Auch dieses Regelungsziel lässt sich dank der Erfindung leichter erreichen.

In einer bevorzugten Ausgestaltung umfasst die Versorgungs-Anordnung eine pneumatische Steuerleitung. Auf der einen Seite ist die pneumatische Steuerleitung mit dem Druckminderer pneumatisch verbunden, auf der anderen Seite mit dem ersten Kanal, und zwar in einem Abzweigungspunkt im ersten Kanal. Bei dieser Ausgestaltung fungiert der Abzweigungspunkt als der Referenzpunkt im ersten Kanal. Die pneumatische Steuerleitung stellt eine Fluidverbindung zwischen dem ersten Kanal, genauer gesagt zwischen dem Abzweigungspunkt, einerseits und den Druckminderer andererseits her. Die Fluidverbindung führt zu einem Druckausgleich zwischen den beiden Enden der pneumatischen Steuerleitung, natürlich in der Regel mit einer gewissen zeitlichen Verzögerung. Dank der Fluidverbindung, die die pneumatische Steuerleitung herstellt, folgt der zeitliche Verlauf des Drucks in der pneumatischen Steuerleitung dem zeitlichen Verlauf des Drucks im Abzweigungspunkt. Bevorzugt bewirkt die pneumatische Steuerleitung einen Druckausgleich zwischen dem Druck im Abzweigungspunkt und dem Druck in einem Bereich des Druckminderers, der mit der pneumatischen Steuerleitung verbunden ist. Falls der Druck im Abzweigungspunkt ausreichend lange konstant ist, so stellt sich dank des Druckausgleichs in diesem Bereich des Druckminderers nach einiger Zeit der gleiche Druck wie im Abzweigungspunkt ein.

Die pneumatische Steuerleitung ist gemäß dieser Ausgestaltung pneumatisch mit dem Druckminderer verbunden. Der Druckminderer vermag zu bewirken, dass der zeitliche Verlauf des Drucks am Hinterdruck-Ausgang dem zeitlichen Verlauf des Drucks in der pneumatischen Steuerleitung folgt. Insgesamt wird dadurch bewirkt, dass der zeitliche Verlauf des Drucks am Hinterdruck-Ausgang dem zeitlichen Verlauf des Drucks im Abzweigungspunkt folgt.

Die Ausgestaltung mit der pneumatischen Steuerleitung erspart in vielen Fällen die Notwendigkeit, dass ein elektronisches Steuergerät einen Bestandteil des Druckminderers ansteuert. Weiterhin ist es zwar möglich, aber dank der Ausgestaltung in vielen Fällen nicht erforderlich, dass ein Drucksensor den Druck am Referenzpunkt oder an einem sonstigen Messpunkt im ersten Kanal misst und das Steuergerät den Druckminderer abhängig von einem Signal des Drucksensors ansteuert. Vielmehr kann der Druckminderer als rein mechanisches und pneumatisches Bauteil ausgestaltet sein. Dies ermöglicht in vielen Fällen eine besonders robuste Ausgestaltung des Druckminderers. Der Druckminderer braucht in einer Ausgestaltung nicht mit elektrischer Energie versorgt zu werden. Auch dann, wenn der Druck, mit dem die zweite Quelle den zweiten Gas-Bestandteil bereitstellt, um ein Mehrfaches größer ist als der Druck, mit dem die erste Quelle den ersten Gas-Bestandteil bereitstellt, vermag in vielen Fällen der pneumatisch arbeitende Druckminderer an seinem Hinterdruck-Ausgang den zweiten Gas-Bestandteil bereitzustellen, ohne dass im Druckminderer sehr große Kräfte oder im zweiten Kanal oder an der Patienten seitigen Koppeleinheit ein sehr großer Druck auftreten.

In einer Fortbildung der Ausgestaltung mit der pneumatischen Steuerleitung weist der Druckminderer im Inneren eine Vordruck-Kammer, eine Hinterdruck-Kammer und eine Steuerdruck-Kammer auf. Unter dem Begriff "Kammer" wird ein Raum verstanden, der allseits fluiddicht umschlossen ist, und zwar bis auf optionale konstruktionsbedingte Öffnungen und in der Regel unvermeidliche Schlitze und Löcher. Möglich ist, dass ein Gehäuse des Druckminderers mindestens eine Wand einer solchen Kammer bildet. Die drei Kammern im Inneren des Druckminderers sind voneinander fluiddicht getrennt, und zwar bis auf optionale konstruktionsbedingte Öffnungen und unvermeidliche Schlitze.

Die Vordruck-Kammer ist über den Vordruck-Eingang mit der ersten Quelle verbunden oder lässt sich wenigstens zeitweise mit der ersten Quelle verbinden. Die Hinterdruck-Kammer ist über den Hinterdruck-Ausgang mit dem zweiten Kanal fluiddicht verbunden. In der Regel herrscht in der Vordruck-Kammer der Vordruck, in der Hinterdruck-Kammer der Hinterdruck. Die Steuerdruck-Kammer ist mit der pneumatischen Steuerleitung verbunden oder steht in einer Fluidverbindung mit der pneumatischen Steuerleitung. Durch diese Verbindung wird Folgendes bewirkt: Der Druck in der Steuerdruck-Kammer folgt dem Druck in der pneumatischen Steuerleitung. Der Druckminderer ist so ausgestaltet, dass Folgendes bewirkt wird: Der zeitliche Verlauf des Drucks am Hinterdruck-Ausgang folgt dem zeitlichen Verlauf des Drucks in der Steuerdruck-Kammer.

Nachfolgend wird eine bevorzugte Realisierungsform der Ausgestaltung mit den drei Kammern beschrieben. Gemäß dieser Realisierungsform umfasst der Druckminderer in seinem Inneren eine Trennwand und einen beweglichen Verschluss. In die Trennwand ist eine Öffnung eingelassen. Der Verschluss vermag die Öffnung in der Trennwand wahlweise freizugeben oder zu verschließen. Die Trennwand trennt die Vordruck-Kammer von der Hinterdruck-Kammer. Bei verschlossener Öffnung trennt die Trennwand die beiden Kammern fluiddicht voneinander. Daher können in den beiden Kammern unterschiedliche Drücke herrschen, und zwar idealerweise, ohne dass ein Druckausgleich stattfindet. Bei freigegebener Öffnung ist eine Fluidverbindung zwischen der Vordruck-Kammer und der Hinterdruck-Kammer hergestellt, so dass ein Druckausgleich stattfindet. In der Regel ist der Druck in der Vordruck-Kammer größer als der Druck in der Hinterdruck-Kammer. Falls eine Flugverbindung zwischen den beiden Kammern hergestellt ist und in den beiden Kammern unterschiedliche Drücke herrschen, fließt der zweite Gas-Bestandteil von der zweiten Quelle durch die Vordruck- und die Hinterdruck-Kammer hindurch in den zweiten Kanal. Bei freigegebener Öffnung findet daher bis zu einem gewissen Grad ein Druckausgleich zwischen den beiden Kammern statt. Diese Fluidverbindung ist bei verschlossener Öffnung unterbrochen, und der zweite Kanal ist dadurch von der zweiten Quelle getrennt. Ein hoher Druck in der zweiten Quelle wirkt sich dann nicht auf die Hinterdruck-Kammer und damit nicht auf den zweiten Kanal aus.

Der Druckminderer ist wie folgt ausgestaltet: Der Verschluss gibt die Öffnung dann frei, wenn ein vorgegebenes Kriterium erfüllt ist. Dieses Kriterium hängt vom Druck in der Steuerdruck-Kammer und / oder vom Druck in der Hinterdruck-Kammer ab. Beispielsweise ist das Kriterium erfüllt, wenn die Differenz zwischen dem Druck in der Steuerdruck-Kammer und den Druck in der Hinterdruck-Kammer oberhalb einer konstruktionsbedingt vorgegebenen Druckdifferenz-Schranke liegt. Oder das Kriterium ist vorgegeben, wenn der Druck in der Steuerdruck-Kammer oberhalb einer konstruktionsbedingt vorgegebenen Druckschranke liegt. Solange das Kriterium nicht erfüllt ist, verschließt der Verschluss die Öffnung. Beispielsweise hält eine Feder oder ein sonstiges Rückstellelement den Verschluss in der verschließenden Position, solange das Kriterium nicht erfüllt ist. Das Kriterium hängt also bevorzugt von der Konstruktion des Druckminderers ab.

In einer Fortbildung der Realisierungsform mit der Trennwand umfasst der Druckminderer zusätzlich zur Trennwand eine bewegliche und / oder flexible Wand. Die bewegliche Wand kann insbesondere eine starre Wand sein, die verschiebbar oder verschwenkbar angeordnet ist, oder eine Wand aus einem biegsamen Material sein, beispielsweise eine Membrane sein. Diese bewegliche oder flexible Wand trennt die Hinterdruck-Kammer von der Steuerdruck-Kammer, und zwar bevorzugt fluiddicht. Bevorzugt ist in die bewegliche oder flexible Wand keine Öffnung eingelassen, so dass kein Fluid vom ersten Kanal durch den Druckminderer hindurch in den zweiten Kanal fließen kann, was oft unerwünscht ist.

Weil diese Wand beweglich ist, und zwar relativ zu einem Gehäuse des Druckminderer, oder flexibel ist, sind das Volumen der Hinterdruck-Kammer und das Volumen der Steuerdruck-Kammer veränderbar. Ein Druckunterschied zwischen den Drücken in den beiden Kammern bewirkt eine Bewegung der beweglichen oder flexiblen Wand, und zwar dergestalt, dass das Volumen der einen Kammer vergrößert und das Volumen der anderen Kammer entsprechend verkleinert wird. Dadurch wird bis zu einem gewissen Grad ein Druckausgleich bewirkt. Diese bewegliche oder flexible Wand steht in einer Wirkverbindung mit dem Verschluss für die Öffnung in der Trennwand, bevorzugt in einer rein mechanischen Wirkverbindung. Dank dieser Wirkverbindung wird eine Bewegung der beweglichen oder flexiblen Wand auf den Verschluss übertragen und führt dazu, dass der Verschluss bewegt wird. Diese Bewegung bewirkt, dass die Öffnung freigegeben oder verschlossen wird.

Die gerade beschriebene Ausgestaltung mit der pneumatischen Steuerleitung bewirkt auf pneumatischem Wege, dass der Druck am Hinterdruck-Ausgang des Druckminderer dem Druck am Referenzpunkt im ersten Kanal folgt. Die nachfolgend beschriebene alternative Ausgestaltung lässt sich mit der pneumatischen Steuerleitung kombinieren oder anstelle einer pneumatischen Steuerleitung verwenden. Gemäß dieser alternativen Ausgestaltung umfasst die Versorgungs-Anordnung einen Drucksensor und ein signalverarbeitendes Druckminderungs-Steuergerät. Möglich ist, dass dasjenige Steuergerät, das das erste Ventil und / oder das zweite Ventil ansteuert, zusätzlich als das Druckminderungs-Steuergerät verwendet wird. Möglich ist auch, dass ein weiteres Steuergerät verwendet wird.

Der Drucksensor vermag ein Maß für den Druck an einem Messpunkt im ersten Kanal zu messen. Bevorzugt fungiert dieser Messpunkt als der Referenzpunkt. Möglich ist auch, dass gesehen in die Fließrichtung ein Abstand zwischen dem Messpunkt und dem Referenzpunkt auftritt und der Druck am Referenzpunkt aus dem gemessenen Druck am Messpunkt und dem Abstand zwischen dem Messpunkt und dem Referenzpunkt hergeleitet wird. Abhängig von Messwerten erzeugt der Drucksensor ein Signal für den Druck am Referenzpunkt. Das Druckminderungs-Steuergerät empfängt und verarbeitet dieses Signal. Abhängig vom empfangenen Signal vermag der Druckminderungs-Steuergerät zu bewirken, dass der zeitliche Verlauf des Drucks am Hinterdruck-Ausgang dem zeitlichen Verlauf des Drucks am Referenzpunkt im ersten Kanal folgt. Das Druckminderungs-Steuergerät wendet bevorzugt hierfür einen geeigneten Regelalgorithmus an.

In einer Fortbildung der Ausgestaltung mit dem Druckminderungs-Steuergerät weist der Druckminderer in seinem Inneren zwei Kammern auf, nämlich eine Vordruck-Kammer und eine Hinterdruck-Kammer. Die Vordruck-Kammer ist über den Vordruck-Eingang mit der ersten Quelle verbunden oder lässt sich wenigstens zeitweise mit der ersten Quelle verbinden. Die Hinterdruck-Kammer ist über den Hinterdruck-Ausgang fluiddicht mit dem zweiten Kanal verbunden. Gemäß dieser Fortbildung umfasst der Druckminderer ein Druckminderer-Stellglied, beispielsweise eine Pumpe oder eine Kolben-Zylinder-Einheit oder ein Bauteil mit einem Hubmagneten und einer Feder einer Feder oder sonstigem Rückstellelement. Das Druckminderungs-Steuergerät vermag das Druckminderer-Stellglied anzusteuern, und zwar abhängig von einem Signal des Drucksensors. Das Druckminderer-Stellglied vermag den Druck in der Hinterdruck-Kammer zu verändern, bevorzugt indem das Stellglied bewirkt, dass das Volumen der Hinterdruck-Kammer verändert wird. Indem es das Druckminderer-Stellglied ansteuert, vermag das Druckminderungs-Steuergerät automatisch zu bewirken, dass der zeitliche Verlauf des Drucks in der Hinterdruck-Kammer dem zeitlichen Verlauf des Drucks am Referenzpunkt im ersten Kanal folgt.

Die Realisierungsform mit dem Drucksensor und dem Druckminderer-Stellglied bewirkt in vielen Fällen, dass der Druck in der Hinterdruck-Kammer besonders rasch dem Druck im ersten Kanal folgt.

Die Realisierungsform mit der pneumatischen Steuerleitung und die Realisierungsform mit dem Druckminderungs-Steuergerät und dem Druckminderer-Stellglied lassen sich miteinander kombinieren. Zum einen wird dadurch Redundanz erzielt. Zum anderen wird dadurch in vielen Fällen bewirkt, dass der zeitliche Verlauf des Drucks am Hinterdruck-Ausgang schneller dem zeitlichen Verlauf des Drucks am Referenzpunkt folgt, als wenn nur die pneumatische Steuerleitung oder nur das Druckminderer-Stellglied vorhanden wären.

In einer Realisierungsform dieser Fortbildung umfasst der Druckminderer eine Trennwand zwischen der Vordruck-Kammer und der Hinterdruck-Kammer, eine Öffnung in dieser Trennwand und einen Verschluss. Mögliche Realisierungsformen und Vorteile dieser Elemente wurden bereits oben als mögliche Realisierungsformen und Vorteile der Ausgestaltung mit der pneumatischen Steuerleitung und der verschließbaren Trennwand beschrieben. Das ansteuerbare Druckminderer-Stellglied steht in einer mechanischen Wirkverbindung mit dem Verschluss. Das angesteuerte Druckminderer-Stellglied vermag den Verschluss zu bewegen und dadurch die Öffnung wahlweise freizugeben oder zu verschließen.

In einer Realisierungsform ist im Inneren des Druckminderers eine bewegliche Wand angeordnet. Diese Wand ist relativ zu einer weiteren Wand der Hinterdruck-Kammer, beispielsweise zu einem Gehäuse des Druckminderers, beweglich. Bevorzugt bildet diese bewegliche Wand eine Wand der Hinterdruck-Kammer. Eine Bewegung der beweglichen Wand bewirkt, dass sich das Volumen der Hinterdruck-Kammer verändert. Dadurch wird auch der Druck in der Hinterdruck-Kammer verändert. Das Druckminderer-Stellglied steht in einer mechanischen Wirkverbindung mit der beweglichen Wand. Indem das Druckminderer-Stellglied die bewegliche Wand bewegt, verändert das Druckminderer-Stellglied den Druck in der Hinterdruck-Kammer und damit den Druck am Hinterdruck-Ausgang des Druckminderers. Anstelle einer beweglichen Wand kann auch eine flexible Wand, beispielsweise eine Membrane, Im Inneren des Druckminderers verwendet werden.

Diese Ausgestaltung ermöglicht auf relativ zuverlässige Weise, dass das Druckminderungs-Steuergerät dem Druck am Hinterdruck-Ausgang regelt. Das Regelungsziel bei dieser Regelung ist, dass der Druck am Hinterdruck-Ausgang dem zeitlichen Verlauf des Drucks am Referenzpunkt folgt. Indem das Druckminderungs-Steuergerät das Druckminderer-Stellglied ansteuert, verändert das Druckminderungs-Steuergerät das Volumen und damit den Druck in der Hinterdruck-Kammer und damit am Hinterdruck-Ausgang.

In einer Ausgestaltung ist im Inneren des Druckminderers zusätzlich eine Steuerdruck-Kammer ausgebildet. Das Druckminderer-Stellglied vermag den Druck in der Steuerdruck-Kammer zu verändern. Der Druck, mit dem dieser Druckminderer an seinem Hinterdruck-Ausgang den zweiten Gas-Bestandteil bereitstellt, hängt wie folgt vom Druck in der Steuerdruck-Kammer ab: Der Druck am Hinterdruck-Ausgang ist umso größer, je größer der Druck in der Steuerdruck-Kammer ist.

Die Ausgestaltung mit der Steuerdruck-Kammer ermöglicht es in vielen Fällen, das Steuerdruck-Stellglied oder wenigstens jedes mechanische Element des Stellglieds vollständig in der Steuerdruck-Kammer anzuordnen. Dadurch ist die oder jede andere Kammer des Druckminderers frei vom Stellglied. Bis zu einem gewissen Grad schützt die Steuerdruck-Kammer das Stellglied vor mechanischen Einflüssen von außen.

Die Erfindung betrifft weiterhin ein Versorgungs-System, welches eine patientenseitige Koppeleinheit mit einem Gasgemisch zu versorgen vermag. Das Gasgemisch umfasst einen ersten Gas-Bestandteil und einen zweiten Gas-Bestandteil. Das Versorgungs-System umfasst eine erste Quelle, eine zweite Quelle und eine erfindungsgemäße Versorgungs-Anordnung.

Die erste Quelle vermag den ersten Gas-Bestandteil bereitzustellen. Die zweite Quelle vermag den zweiten Gas-Bestandteil bereitzustellen. Zwischen dem Versorgungs-Verbindungselement des ersten Kanals der Versorgungs-Anordnung und der ersten Quelle ist wenigstens zeitweise eine Fluidverbindung hergestellt oder lässt sich herstellen. Zwischen dem Vordruck-Eingang des Druckminderers der Versorgungs-Anordnung und der zweiten Quelle ist ebenfalls wenigstens zeitweise eine Fluidverbindung hergestellt oder lässt sich herstellen.

In einer Ausgestaltung stellt die zweite Quelle den zweiten Gas-Bestandteil mit einem höheren Druck bereit als die erste Quelle den ersten Gas-Bestandteil. Beispielsweise ist die zweite Quelle eine stationäre Quelle, die mit einem Versorgungsnetz einer Infrastruktur verbunden ist, oder umfasst mindestens eine unter Druck stehenden Flasche. Die erste Quelle ist beispielsweise ein Einlass einer Fluidförderereinheit, insbesondere eines Gebläses oder einer Pumpe, wobei die Fluidförderereinheit beispielsweise zu einem Beatmungsgerät gehört. Die erste Quelle kann auch eine sonstige mobile Quelle sein.

Weiterhin betrifft die Erfindung ein Beatmungs-System zur künstlichen Beatmung eines Patienten, wobei der Patient mit einem Gasgemisch umfassend einen ersten Gas-Bestandteil und einen zweiten Gas-Bestandteil beatmet wird. Mindestens einer der beiden Gas-Bestandteile, optional beide Gas-Bestandteile, ist oder enthält Sauerstoff. Das Gasgemisch kann einen dritten Gas-Bestandteil umfassen, beispielsweise ein Narkosemittel.

Das Beatmungs-System umfasst
- eine Fluidförderereinheit, beispielsweise ein Gebläse oder eine Pumpe,
- eine patientenseitige Koppeleinheit sowie
- eine erfindungsgemäße Versorgungs-Anordnung oder ein erfindungsgemäßes Versorgungs-System.

Die patientenseitige Koppeleinheit ist mit einem Patienten verbunden oder lässt sich wenigstens zeitweise mit einem Patienten verbunden, insbesondere im oder am Körper des Patienten anbringen.

Während der künstlichen Beatmung ist zwischen einer ersten Quelle, die den ersten Gas-Bestandteil bereitstellt, und dem Versorgungs-Verbindungselement eine Fluidverbindung hergestellt. Weiterhin ist während der künstlichen Beatmung zwischen einer zweiten Quelle für den zweiten Gas-Bestandteil und dem Vordruck-Eingang des Druckminderers eine Fluidverbindung hergestellt. Die Fluidfördereinheit vermag den ersten Gas-Bestandteil von der ersten Quelle durch den ersten Kanal hindurch zum Mischpunkt zu fördern. Der zweite Gas-Bestandteil fließt von der zweiten Quelle zum Mischpunkt, beispielsweise aufgrund eines ausreichend hohen Drucks in der zweiten Quelle oder durch dieselbe oder eine weitere Fluidfördereinheit. Der zeitliche Verlauf des Drucks am Hinterdruck-Ausgang des Druckminderers folgt dem zeitlichen Verlauf des Drucks am Referenzpunkt im ersten Kanal.

Die beiden Gas-Bestandteile werden im Mischpunkt zum Gasgemisch vermischt oder vermischen sich dort von allein. Das im Mischpunkt erzeugte oder entstandene Gasgemisch fließt durch den Inspirations-Kanal zur patientenseitigen Koppeleinheit.

Die Fluidförderereinheit führt eine Abfolge von Beatmungshüben aus. In jedem Beatmungshub wird eine Menge des Gasgemischs, das im Mischpunkt erzeugt wird oder entsteht, durch den Inspirations-Kanal zu der patientenseitigen Koppeleinheit gefördert. Die Fluidfördereinheit ist oder umfasst beispielsweise eine Pumpe oder eine Kolben-Zylinder-Einheit oder ein Gebläse, das im Zusammenwirken mit mindestens einem Ventil arbeitet, oder auch ein Handbeatmungsbeutel.

In einer Anwendung führt der Patient während der künstlichen Beatmung eine eigene Atmungsaktivität aus, insbesondere aufgrund seiner eigenen spontanen Atmung oder weil seine Atmungsmuskulatur extern stimuliert wird. Bei dieser Anwendung wird bevorzugt die Fluidförderereinheit derart angesteuert, dass jeder Atemzug des Patienten einen Beatmungshub der Fluidförderereinheit auslöst und ein Beatmungshub nur als Reaktion auf einen Atemzug ausgeführt wird. Mindestens ein Sensor erzeugt hierbei ein respiratorisches Signal, welches ein Maß für die eigene Atmungsaktivität des Patienten ist. Die künstliche Beatmung unterstützt also die eigene Atmungsaktivität des Patienten. Idealerweise sind die Beatmungshübe, welche die Fluidfiltereinheit ausführt, ideal mit der eigenen Atmungsaktivität des Patienten synchronisiert.

In einer Ausgestaltung steht die Fluidfördereinheit in einer Fluidverbindung mit dem ersten Kanal und speist den ersten Gas-Bestandteil in den ersten Kanal ein. Bevorzugt ist dieser erste Gas-Bestandteil Umgebungsluft oder ein Gas, welches in einem Beatmungskreislauf vom Patienten zur Fluidfördereinheit geleitet wird. Ein stationärer oder mobiler Versorgungsanschluss speist den zweiten Gas-Bestandteil in den zweiten Kanal ein.

Im Folgenden wird die Erfindung anhand eines Ausführungsbeispiels beschrieben. Hierbei zeigt
- Figur 1: schematisch, wie ein Patient mit einem Gasgemisch aus Luft und Sauerstoff künstlich beatmet wird, wobei zwei Kanäle die beiden Bestandteile liefern;
- Figur 2: schematisch einen Beatmungskreislauf, in dem ein Patient mit einem Gemisch aus Luft, Sauerstoff und einem Narkosemittel versorgt und narkotisiert wird;
- Figur 3: den zeitlichen Verlauf des Volumenflusses und des Drucks des Gasgemisches, das zum Patienten fließt;
- Figur 4: schematisch einen rein pneumatischen Druckminderer;
- Figur 5: eine beispielhafte Abhängigkeit des Hinterdrucks vom Volumenfluss;
- Figur 6: schematisch einen angesteuerten Druckminderer mit einer Steuerdruck-Kammer.
- Figur 7: schematisch einen angesteuerten Druckminderer ohne eine Steuerdruck-Kammer.

Im Ausführungsbeispiel wird die Erfindung benutzt, um einen Patienten Pt künstlich zu beatmen. Am oder im Körper des Patienten Pt ist eine patientenseitige Koppeleinheit 9 befestigt, beispielsweise eine Atemmaske oder ein Tubus oder ein Katheter.

Ein nur schematisch gezeigtes Beatmungsgerät 100 führt eine Abfolge von Beatmungshüben aus und fördert in jedem Beatmungshub ein Gasgemisch zur patientenseitigen Koppeleinheit 9 und damit zum Patienten Pt. Dieses Gasgemisch enthält einen Anteil (Vol-%) von Sauerstoff, wobei ein Benutzer diesen Anteil vorgegeben hat. Dieser Sauerstoff-Anteil kann oberhalb des Anteils von Sauerstoff in der Atemluft liegen. Um den Anteil von Sauerstoff gegenüber der Atemluft zu vergrößern, wird im Ausführungsbeispiel ein Gasgemisch aus Atemluft und reinem Sauerstoff erzeugt. Das Gasgemisch kann zusätzlich ein Narkosemittel enthalten, sodass der Patient Pt sediert oder narkotisiert wird.

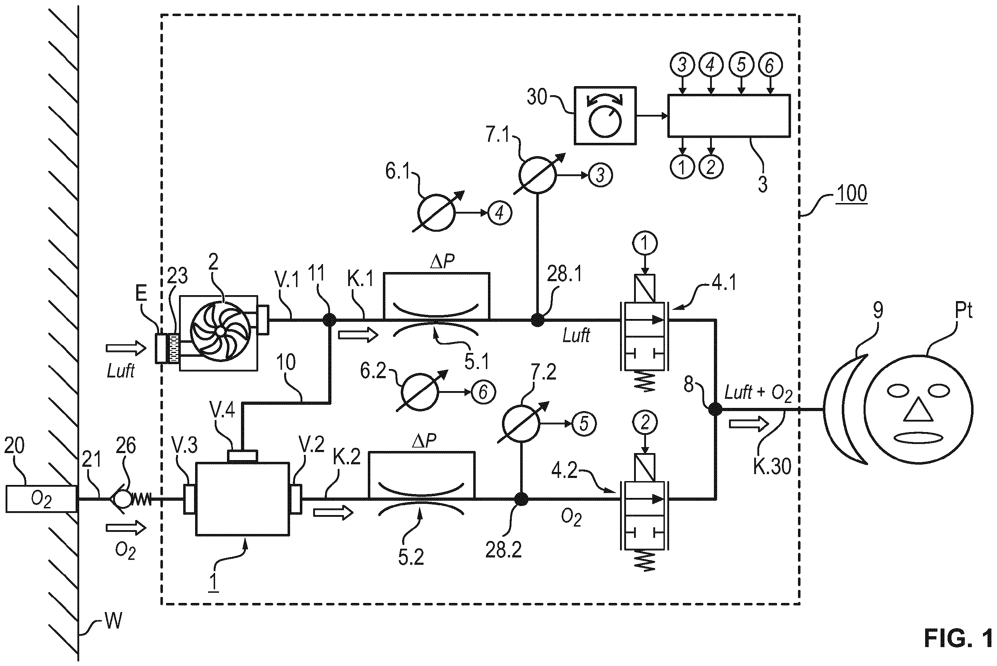

Figur 1 zeigt schematisch eine Anordnung, um dieses Gasgemisch mit einem höheren Sauerstoffanteil zu erzeugen. Die beiden Bestandteile des Gasgemischs, nämlich Luft und reiner Sauerstoff, werden in zwei Kanälen K.1 und K.2 bereitgestellt, wobei ein erster Kanal K.1 die Luft und ein zweiter Kanal K.2 den Sauerstoff bereitstellt und wobei die beiden Kanäle K.1 und K.2 in einem Mischpunkt 8 zusammengeführt werden.

Möglich ist, dass ein Gasmischer die beiden Gas-Bestandteile zum Gasgemisch zusammenmischt. Ein solcher Gasmischer wird beispielsweise in DE 102008057180 B3, in DE 102012008108 A1 und in DE 102016001383 A1 beschrieben. Im einfachsten Falle ist im Mischpunkt 8 ein Y-Stück angeordnet.

Von diesem Mischpunkt 8 führt ein Inspirations-Kanal K.30, beispielsweise ein Schlauch für die Einatmung und optional ein Zwei-Lumen-Schlauch mit einem Lumen für die Einatmung und einem Lumen für die Ausatmung, zur patientenseitigen Koppeleinheit 9. Dieser Inspirations-Kanal K.30 leitet das Gemisch aus Luft und reinem Sauerstoff zur patientenseitigen Koppeleinheit 9.

Ein Benutzer gibt einen gewünschten Sauerstoff-Anteil im Gasgemisch vor. Beispielsweise stellt der Benutzer manuell den gewünschten Sauerstoffgehalt an einem schematisch gezeigten Drehknopf 30 ein.

Ein Gebläse 2 oder eine sonstige Förderereinheit des Beatmungsgeräts 100 saugt Umgebungsluft durch einen Einlass E des Beatmungsgeräts 100 an und speist die angesaugte Luft in den ersten Kanal K.1 ein. Zwischen dem Einlass E und dem Gebläse 2 ist ein Filter 23 angeordnet. In der Anwendung gemäß Figur 1 fungiert die Förderereinheit 2 als die Quelle für den ersten Gas-Bestandteil.

Ein Eingang des ersten Kanals K.1 in Form eines Versorgungs-Verbindungselements V.1 ist mit einem Versorgungs-Ausgang des Gebläses 2 verbunden. Der Druck im ersten Kanal K.1 folgt idealerweise einem vorgegebenen zeitlichen Verlauf, ist beispielsweise zeitlich konstant. Der Druck im ersten Kanal K.1 liegt im Ausführungsbeispiel oberhalb des maximalen Beatmungsdrucks, also oberhalb des maximalen Drucks, mit dem das Gasgemisch zur patientenseitigen Koppeleinheit 9 und weiter in die Lunge des Patienten Pt gefördert wird, und liegt bevorzugt zwischen 20 mbar und 100 mbar.

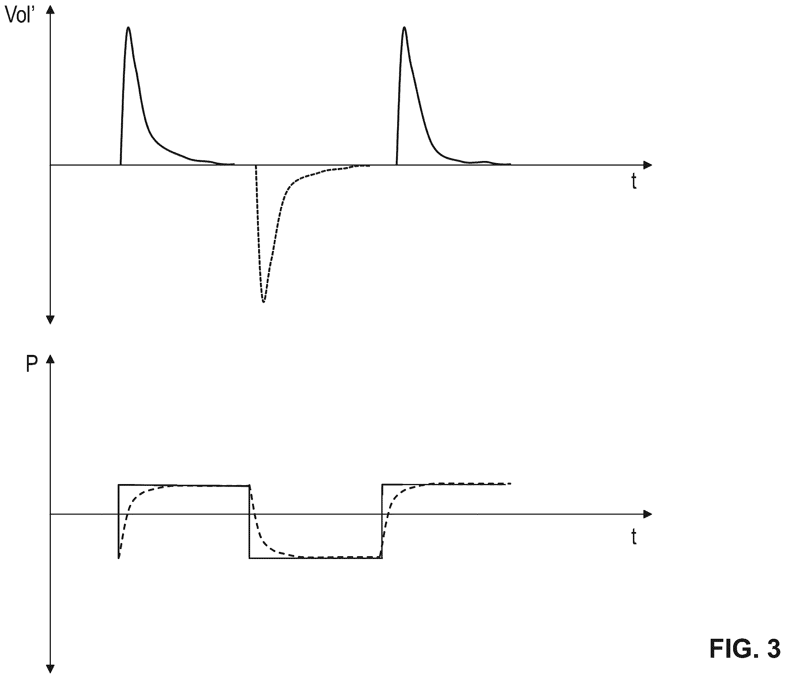

Der Volumenfluss, also der Fluss von Gas pro Zeiteinheit, durch den ersten Kanal K.1 flussabwärts von der Förderereinheit 2 und / oder der Druck im ersten Kanal K.1 sollen jeweils einem vorgegebenen zeitlichen Verlauf folgen. Figur 3 zeigt oben einen beispielhaften geforderten zeitlichen Verlauf des Volumenflusses (Vol') und unten einen beispielhaften geforderten zeitlichen Verlauf des Drucks (P). Werte oberhalb der x-Achse bedeuten einen Fluss des Gasgemischs zum Patienten Pt hin (Einatmung, Inspiration), Werte unterhalb einen Fluss vom Patienten Pt weg (Ausatmung, Exspiration).

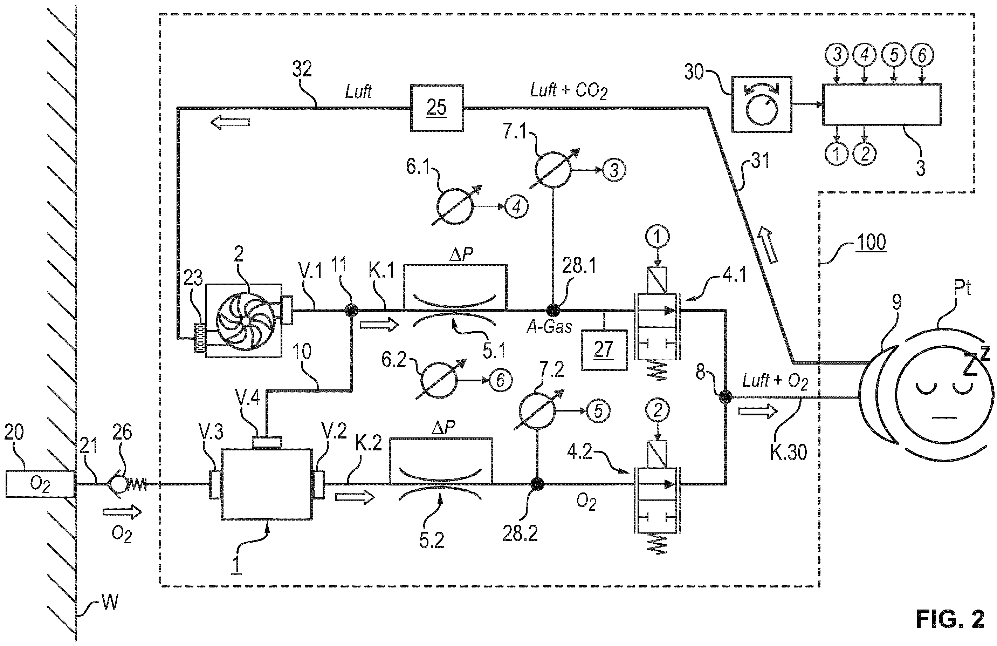

Ein signalverarbeitendes Steuergerät 3 führt eine Regelung durch, wobei der tatsächliche Volumenfluss die Regelgröße und der vorgegebene zeitliche Verlauf des Volumenflusses die Führungsgröße ist, vgl. Figur 1 und Figur 2. Das Steuergerät 3 steuert ein Proportionalventil 4.1 an und verändert dadurch den Volumenfluss im ersten Kanal K.1 flussabwärts vom Proportionalventil 4.1 zum Mischpunkt 8.

Im ersten Kanal K.1 ist ein pneumatischer Widerstand 5.1, beispielsweise eine Engstelle, angeordnet. Ein Volumenfluss-Sensor 6.1 misst die Differenz ΔP zwischen dem Druck flussaufwärts und dem Druck flussabwärts vom pneumatischen Widerstand 5.1 und leitet aus der Druckdifferenz ein Maß für den tatsächlichen Volumenfluss durch den ersten Kanal K.1 her. Außerdem misst ein Drucksensor 7.1 den tatsächlichen Druck im ersten Kanal K.1 an einem Messpunkt 28.1 flussabwärts vom pneumatischen Widerstand 5.1. Das Steuergerät 3 erhält jeweils ein Signal von den beiden Sensoren 6.1 und 7.1.

Der zweite Kanal K.2 erhält reinen Sauerstoff (O2) aus einer Versorgungsleitung 21, die in Verbindung mit einem Versorgungsanschluss 20 steht. Im gezeigten Beispiel ist dieser Versorgungsanschluss 20 stationär in einer Wand W angeordnet und gehört zu einem stationären Versorgungssystem einer Krankenhaus-Infrastruktur. Möglich ist auch, dass der zweite Kanal K.2 reinen Sauerstoff aus mindestens einer unter Druck stehenden Flasche erhält. Der Versorgungsanschluss 20 stellt den reinen Sauerstoff mit einem Druck bereit, der zwischen 2 bar und 8 bar liegt. Ein optionales Rückschlagventil 26 in der Versorgungsleitung 21 verhindert, dass reiner Sauerstoff zurück in den Versorgungsanschluss 20 und dann in die Krankenhaus-Infrastruktur gepresst wird.

Ein pneumatischer Druckminderer 1 umfasst einen Vordruck-Eingang V.3 und einen Hinterdruck-Ausgang V.2. Der Vordruck-Eingang V.3 ist mit der Versorgungsleitung 21 verbunden, der Hinterdruck-Ausgang V.2 mit dem zweiten Kanal K.2. Der Druckminderer 1 reduziert den Druck vom Versorgungsanschluss 20 mit dem Ziel, dass der Druck am Hinterdruck-Ausgang V.2 des Druckminderers 1 dem zeitlich veränderlichen Druck an einem nachfolgend beschriebenen Referenzpunkt 11, 28.1 folgt. Dadurch folgt der Druck am Hinterdruck-Ausgang V.2 dem Druck am Versorgungs-Verbindungselement V.1 und somit dem Druck am Versorgungs-Ausgang des Gebläses 2. Wie dieses Ziel erreicht wird, wird weiter unten eingehender beschrieben.

Im zweiten Kanal K.2 sind ein pneumatischer Widerstand 5.2, ein Volumenfluss-Sensor 6.2, ein Drucksensor 7.2 und ein Proportionalventil 4.2 angeordnet. Diese Bauteile funktionieren so wie die entsprechenden Bauteile im ersten Kanal K.1. Der Drucksensor 7.2 misst an einem Messpunkt 28.2 ein Maß für den Druck im zweiten Kanal K.2. Das Steuergerät 3 steuert Im Ausführungsbeispiel das Proportionalventil 4.2 mit dem Regelungsziel an, dass der tatsächliche Volumenfluss durch den zweiten Kanal K.2 einem vorgegebenen zeitlichen Verlauf folgt.

Figur 2 zeigt eine weitere Anwendung der Erfindung, bei welcher der Patient Pt nicht nur künstlich beatmet, sondern zusätzlich sediert oder narkotisiert wird. Gleiche Bezugszeichen haben die gleiche Bedeutung wie in Figur 1.

Die Ausgestaltung gemäß Figur 2 hat folgende zusätzlichen Bestandteile:
- einen Narkosemittelverdampfer 27, der gasförmiges Narkosemittel erzeugt,
- eine Exspirations-Fluidverbindung mit einem ersten Abschnitt 31 und einem zweiten Abschnitt 32 und
- einen Absorber 25 für CO2 und Narkosemittel.

Das gasförmige Narkosemittel, welches der Narkosemittelverdampfer 27 erzeugt hat, wird in das Gasgemisch eingespeist, welches durch den Inspirations-Kanal K.30 hindurch zur patientenseitigen Koppeleinheit 9 geleitet und vom Patienten Pt eingeatmet wird. Im gezeigten Beispiel wird das Narkosemittel in den ersten Kanal K.1 eingespeist und fließt in diesen zum Mischpunkt 8. Möglich ist auch, dass es in den zweiten Kanal K.2 oder in die Inspirations-Kanal K.30 eingespeist wird.

Die vom Patienten Pt ausgeatmete Luft enthält häufig noch Narkosemittel. Dieses Narkosemittel soll nicht in die Umgebung austreten. Daher wird ein Beatmungskreislauf gebildet. Die Exspirations-Fluidverbindung 31, 32 führt von der patientenseitigen Koppeleinheit 9 zurück zum Gebläse 2. Das Gebläse 2 hält einen Fluss von Gas durch diesen Beatmungskreislauf aufrecht. Der erste Abschnitt 31 führt von der patientenseitigen Koppeleinheit zum CO2-Absorber 25. Dieser CO2-Absorber 25 absorbiert Kohlendioxid und optional auch Narkosemittel aus der ausgeatmeten Luft. Der zweite Abschnitt 32 führt die ausgeatmete Luft, aus der das Kohlendioxid und optional das Narkosemittel absorbiert wurden, zum Filter 23 und von dort zum Gebläse 2.

In dieser Ausgestaltung fungiert der Absorber 25 als die Quelle für den ersten Gas-Bestandteil (Luft), die in den ersten Kanal K.1 gelangt.

Wie bereits dargelegt, wird ein Gasgemisch vom Mischpunkt 8 zur patientenseitigen Koppeleinheit 9 gefördert. Der tatsächliche Volumenfluss dieses Gasgemischs flussabwärts vom Mischpunkt 8 soll einem vorgegebenen zeitlichen Verlauf folgen. Außerdem ist ein geforderter Anteil von Sauerstoff vorgegeben, bevorzugt als Vol-% vorgegeben. Dieser Sauerstoffanteil kann zeitlich konstant oder zeitlich variabel sein. Aus dem vorgegebenen zeitlichen Verlauf des Volumenflusses zur patientenseitigen Koppeleinheit 9, dem geforderten Anteil von Sauerstoff im Gasgemisch sowie dem bekannten Anteil von Sauerstoff in Luft resultieren ein Soll-Verlauf des Volumenflusses durch den ersten Kanal K.1 und ein Soll-Verlauf des Volumenflusses durch den zweiten Kanal K.2. Das Steuergerät 3 oder ein übergeordnetes Steuergerät berechnen diese beiden Soll-Verläufe für die beiden Kanäle K.1 und K.2, und das Steuergerät 3 steuert die beiden Proportionalventile 4.1 und 4.2 abhängig von diesen beiden Soll-Verläufen an. Das Steuergerät 3 führt also zwei Regelungen des Volumenflusses durch, nämlich eine im ersten Kanal K.1 und eine im zweiten Kanal K.2. In vielen Fällen lässt sich derselbe Regelalgorithmus verwenden, um die beiden Proportionalventile 4.1 und 4.2 anzusteuern. Dies ist insbesondere deshalb möglich, weil am Versorgungs-Ausgang des Gebläses 2 und am Hinterdruck-Ausgang V.2 des Druckminderers 1 derselbe Druck herrscht. Dies wird durch die nachfolgend beschriebene pneumatische oder elektronische Steuerung (open-loop control) oder Regelung (closed-loop control) bewirkt.

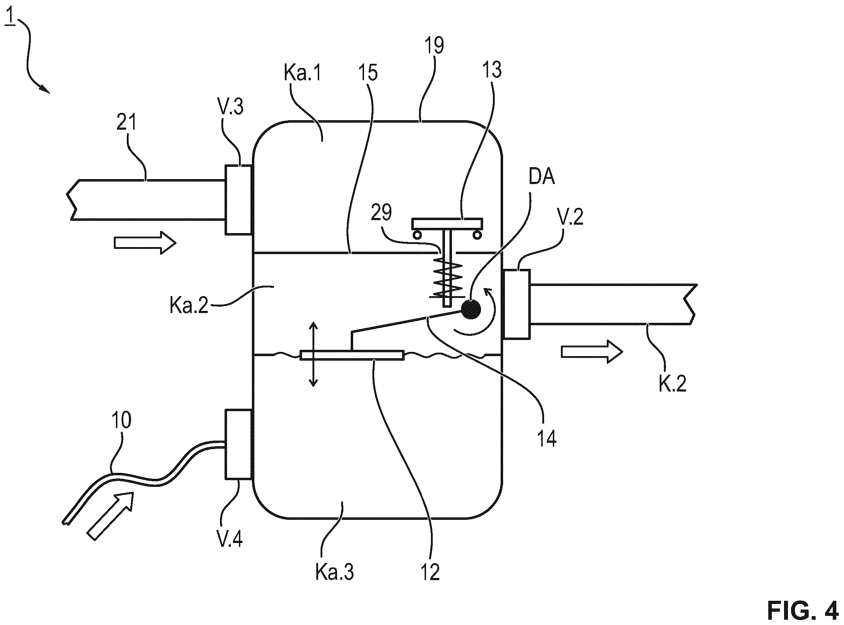

Figur 1 und Figur 4 veranschaulichen eine pneumatische Steuerung des Druckminderers 1. Eine pneumatische Steuerleitung 10 führt von einem Abzweigungspunkt 11 im ersten Kanal K.1 zu einem Steuerdruck-Eingang V.4 des Druckminderers 1. Dieser Steuerdruck-Eingang V.4 wird für die pneumatische Steuerung benötigt, aber nicht für die weiter unten beschriebene elektronische Steuerung oder Regelung. Bevorzugt ist die pneumatische Steuerleitung 10 als flexibler Schlauch ausgelegt, sie kann auch eine starre Röhre sein. Der Abzweigungspunkt 11 ist flussabwärts vom Gebläse 2 und flussaufwärts vom Proportionalventil 4.1 angeordnet, in der gezeigten Realisierung auch flussaufwärts vom pneumatischen Widerstand 5.1. beginnt, in einer Ausgestaltung fungiert dieser Abzweigungspunkt 11 als der Referenzpunkt.

Dank der pneumatischen Steuerleitung 10 liegt am Steuerdruck-Eingang V.4 des Druckminderers 1 - bis auf unvermeidliche zeitliche Verzögerungen und Undichtigkeiten - stets derselbe Druck an wie im ersten Kanal K.1 und dort im Abschnitt zwischen dem Gebläse 2 und dem Proportionalventil 4.1 und insbesondere wie im Referenzpunkt (Abzweigungspunkt 11). Dieser zeitlich veränderliche Druck im ersten Kanal K.1 fungiert als ein Steuerdruck und somit als ein Master, und der Druck im Eingang des zweiten Kanals K.2 folgt als ein Slave diesem zeitlich veränderlichen Steuerdruck.

Figur 4 zeigt eine beispielhafte Ausgestaltung eines pneumatisch arbeitenden Druckminderers 1. Der Vordruck-Eingang V.3 des Druckminderers 1 ist mit der Versorgungsleitung 21 verbunden. Der Steuerdruck-Eingang V.4 ist mit der pneumatischen Steuerleitung 10 verbunden. Der zweite Kanal K.2 beginnt im Hinterdruck-Ausgang V.2 des Druckminderers 1.

Ein starres Gehäuse 19 umschließt das Innere des Druckminderers 1. In diesem Inneren werden drei Kammern gebildet, nämlich
- eine Vordruck-Kammer Ka.1, die über den Vordruck-Eingang V.3 in einer Fluidverbindung mit der Versorgungsleitung 21 steht,
- eine Hinterdruck-Kammer Ka.2, die über den Hinterdruck-Ausgang V.2 in einer Fluidverbindung mit dem zweiten Kanal K.2 steht, sowie
- eine Steuerdruck-Kammer Ka.3, die über den Steuerdruck-Eingang V.4 in einer Fluidverbindung mit der pneumatischen Steuerleitung 10 steht.

Eine Trennwand 15 im Druckminderer 1 trennt die Hinterdruck-Kammer Ka.2 von der Vordruck-Kammer Ka.1. Bevorzugt ist die Trennwand 15 starr. In die Trennwand 15 ist eine Öffnung 29 eingelassen. Ein federbelasteter Verschluss 13 ist relativ zur Trennwand 15 in zwei entgegengesetzte Richtungen linear beweglich (in Figur 3 vertikal nach oben und nach unten) und kann diese Öffnung 29 freigeben oder verschließen.

Eine bewegliche Wand 12 ist innen am Gehäuse des Druckminderers 1 befestigt und trennt die Steuerdruck-Kammer Ka.2 von der Hinterdruck-Kammer Ka.3. Im Ausführungsbeispiel hat die bewegliche Wand 12 die Form einer flexiblen Membrane. In einer Ausgestaltung umfasst die Membrane 12 eine zentriert angeordnete feste Platte. Die bewegliche Wand 12 kann auch die Form einer starren Platte aufweisen, die in beiden Richtungen relativ zum Gehäuse 19 vertikal verschieblich ist. Bevorzugt trennt die bewegliche Wand 12 die beiden Kammern Ka.2 und Ka.3 - bis auf unvermeidliche Undichtigkeiten - fluiddicht voneinander.

Die gerade beschriebene Konstruktion des Druckminderers 1 bewirkt, dass sich in der Vordruck-Kammer Ka.1 und in der Hinterdruck-Kammer Ka.2 der zweite Gas-Bestandteil, hier Sauerstoff, befindet und in der Steuerdruck-Kammer Ka.3 der erste Gas-Bestandteil, hier Luft. Die bewegliche Wand 12 verhindert, dass sich diese beiden Gas-Bestandteile im Druckminderer 1 miteinander mischen. In einem Zustand, in dem der Verschluss 13 die Öffnung 29 freigibt, stehen die beiden Kammern Ka.1 und Ka.2 in einer Fluidverbindung miteinander, und ein Druckausgleich kann stattfinden. Bei verschlossener Öffnung 29 unterbricht die Trennwand 15 diese Fluidverbindung und verhindert einen Druckausgleich.

Ein Hebel 14 ist um eine Drehachse DA drehbar und liegt oben auf der beweglichen Wand 12, optional oben auf der festen Platte der Membrane, auf. Der Verschluss 13 liegt oben auf dem Hebel 14 auf. Wie in Figur 4 zu sehen ist, werden ein kurzer Hebelarm zwischen der Drehachse DA und dem Auflagepunkt des Verschlusses 13 sowie ein langer Hebelarm zwischen der Drehachse DA und dem Auflagepunkt des Hebels 14 auf der beweglichen Wand 12 gebildet. Dadurch steht die bewegliche Wand 12 in einer Wirkverbindung mit dem Verschluss 13 und kann eine große Kraft auf den Verschluss 13 ausüben.

In der Vordruck-Kammer Ka.1 herrscht derselbe Druck wie in der Versorgungsleitung 21, also ein Vordruck, der bevorzugt zwischen 2 bar und 8 bar liegt und damit um ein Vielfaches oberhalb des Drucks in den beiden Kanälen K.1 und K.2. In der Steuerdruck-Kammer Ka.3 herrscht derselbe Druck wie in der der pneumatischen Steuerleitung 10 und somit auch idealerweise derselbe Druck wie im ersten Kanal K.1 und dort im Abschnitt zwischen dem Gebläse 2 und dem Proportionalventil 4.1. In der Hinterdruck-Kammer Ka.2 wird derjenige Druck erzeugt, mit dem der zweite Kanal K.2 reinen Sauerstoff bereitstellt.

Dank der beweglichen Wand 12 stellt sich auch bei einem veränderlichen Steuerdruck (Druck im ersten Kanal K.1) in den beiden Kammern Ka.2 und Ka.3 stets derselbe Druck ein. Nach einer Veränderung des Steuerdrucks, also des Drucks in der Steuerdruck-Kammer Ka.3, verstreicht in der Regel eine Einschwing-Phase, bis die Drücke wieder gleich sind. Je nach der Position der beweglichen Wand 12 öffnet oder verschließt der Verschluss 13 die Öffnung 29 in der Trennwand 15 und ermöglicht oder verhindert somit, dass reiner Sauerstoff aus der Versorgungsleitung 21 durch den Druckminderer 1 hindurch in den zweiten Kanal K.2 fließt. Bei einem ausreichend großen Druck in der Hinterdruck-Kammer Ka.2 bewirkt die bewegliche Wand 12 mittels der Wirkverbindung (Hebel 14), dass der Verschluss 13 die Öffnung 29 verschließt.

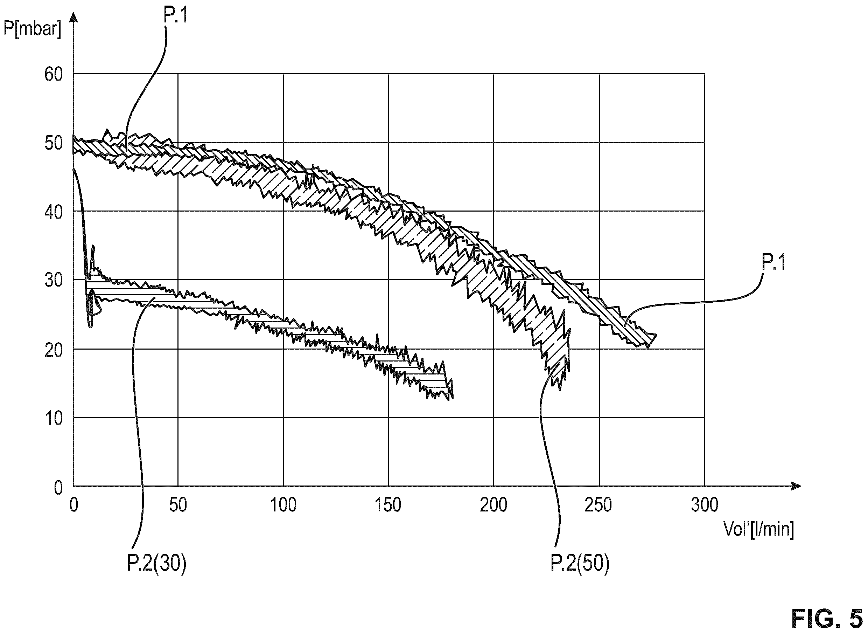

Idealerweise erzeugt das Gebläse 2 einen konstanten Druck, und zwar unabhängig vom Volumenfluss. Idealerweise liegt daher am Ausgang des Druckminderers 1 ebenfalls dieser konstante Druck an. In der Praxis nimmt der erzeugte Druck mit zunehmendem Volumenfluss ab. Figur 5 zeigt diese Situation beispielhaft, wobei auf der x-Achse der Volumenfluss Vol' in [l/min] und auf der y-Achse der erzeugte Hinterdruck P in [mbar] aufgetragen sind. Die Bezeichnung "Hinterdruck" bezieht sich auf den Ausgang des Gebläses 2. Die Kurve P.2(50) zeigt den Verlauf des tatsächlichen Drucks P, den das Gebläse 2 erzeugt, abhängig vom Volumenfluss Vol' in einer Situation, in der das Gebläse 2 einen Druck von 50 mbar erzeugen soll. Die Kurve P.2(30) zeigt den tatsächlichen Druck bei einem Solldruck von 30 mbar. Die Kurve P.1 zeigt den tatsächlichen Druck am Hinterdruck-Ausgang V.2 des Druckminderers 1. Zu sehen ist, dass trotz der Abhängigkeit vom Volumenfluss der Druck am Ausgang des Druckminderers 1 gut mit dem Steuerdruck (Hinterdruck des Gebläses 2) übereinstimmt.

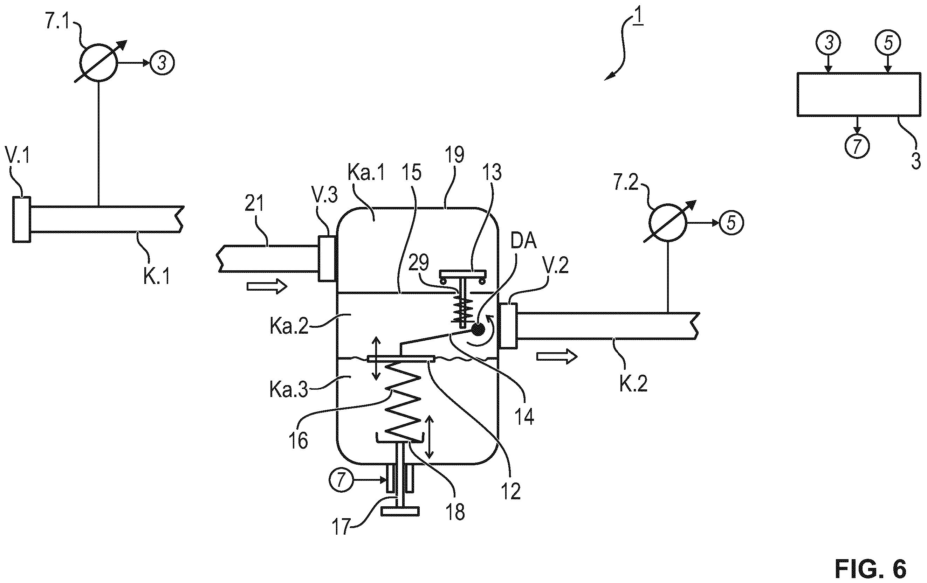

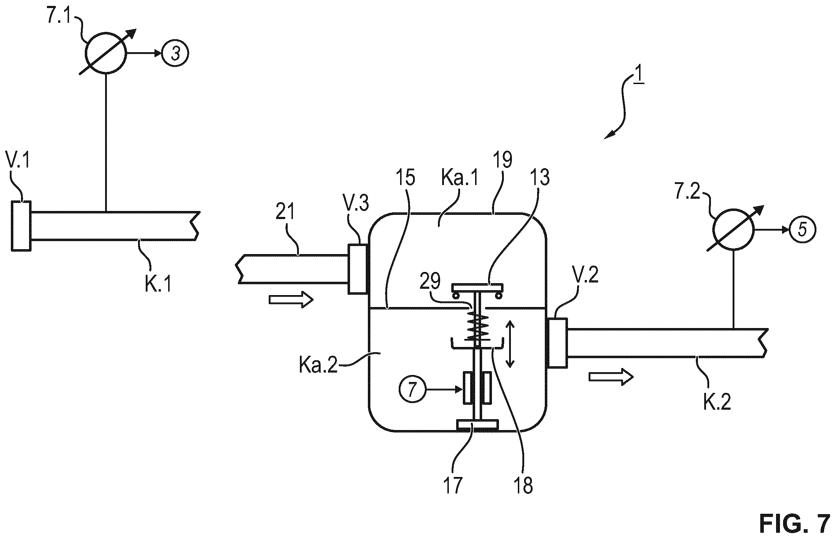

Figur 6 und Figur 7 zeigen zwei alternative Ausgestaltungen des Druckminderers 1. Gleiche Bezugszeichen haben die gleiche Bedeutung wie in Figur 1 und Figur 4. Als Referenzpunkt fungiert der Messpunkt 28.1, in dem der Drucksensor 7.1 den Druck misst.

Die beiden alternativen Ausgestaltungen sparen eine pneumatische Steuerleitung 10 vom ersten Kanal K.1 zum Druckminderer 1 sowie einen pneumatischen Steuerdruck-Eingang V.4 ein. Gleiche Bezugszeichen haben die gleiche Bedeutung wie in Figur 4. Bei beiden alternativen Ausgestaltungen hat der Druckminderer 1 nur einen Eingang, nämlich den Vordruck-Eingang V.3, der wiederum die Vordruck-Kammer Ka.1 mit der Versorgungsleitung 21 verbindet.

Zunächst wird die Ausgestaltung gemäß Figur 6 beschrieben. Auf die bewegliche Wand 12 wirkt wiederum von einer Seite der Druck in der Hinterdruck-Kammer Ka.2 ein, von der anderen Seite die Kraft einer Feder 16 in der Steuerdruck-Kammer Ka.3. Die Feder 16 ist auf der einen Seite mit der beweglichen Wand 12 und auf der anderen Seite mit einem mechanischen Verbindungselement 18 verbunden und ist bestrebt, sich auszudehnen. Ein angesteuertes Steuerdruck-Stellglied 17, beispielsweise eine Pumpe oder eine Kolben-Zylinder-Einheit, stützt sich am Gehäuse 19 des Druckminderers 1 ab und wirkt von einer Seite auf das Verbindungselement 18. Das Steuerdruck-Stellglied 17 vermag den Abstand zwischen dem Verbindungselement 18 und der unteren Wand des Gehäuses 19 und dadurch auch die Kraft der Feder 16 zu vergrößern und zu verkleinern. Je dichter das Verbindungselement 18 der beweglichen Wand 12 ist, desto größer ist die Federkraft der Feder 16.

Das Steuergerät 3 empfängt jeweils ein Signal von den beiden Drucksensoren 7.1 und 7.2, vgl. Figur 1, und steuert das Steuerdruck-Stellglied 17 so an, dass der Druck in der Steuerdruck-Kammer Ka.3 und damit der Druck im zweiten Kanal K.2 dem Druck im ersten Kanal K.1 flussabwärts vom Gebläse 2 folgt.

Der Druckminderer 1 gemäß Figur 7 umfasst keine Hinterdruck-Kammer und keine bewegliche Wand. Das Steuerdruck-Stellglied 17 befindet sich in der Hinterdruck-Kammer Ka.2 und ist über das Verbindungselement 18 mit dem Verschluss 13 mechanisch verbunden. Das Steuerdruck-Stellglied 17 vermag direkt den Verschluss 13 in zwei entgegengesetzte vertikale Richtungen zu bewegen und dadurch den Verschluss 13 wahlweise in eine freigebende oder in eine verschließende Position zu bewegen.

Die beiden Ausgestaltungen gemäß Figur 4 und gemäß Figur 6 oder Figur 7 des Druckminderers 1 lassen sich miteinander kombinieren. Gemäß dieser Kombination weist der Druckminderer 1 also sowohl den Steuerdruck-Eingang V.4 als auch die Feder 16, das Steuerdruck-Stellglied 17 und das Verbindungselement 18 auf. Die Versorgungs-Anordnung ist bei dieser Kombination bevorzugt so wie in Figur 1 gezeigt ausgestaltet. Das angesteuerte Steuerdruck-Stellglied 17 und die Feder 16 können bis zu einem gewissen Grad Unterschiede zwischen dem Druck in der Hinterdruck-Kammer Ka.2 und dem Druck im ersten Kanal K.1 kompensieren.

### Bezugszeichenliste

| | |
|---|---|
| 1 | pneumatischer Druckminderer zwischen der Versorgungsleitung 21 und dem zweiten Kanal K.2, umfasst den Hinterdruck-Ausgang V.2, den Vordruck-Eingang V.3 und den optionalen Steuerdruck-Eingang V.4 |
| 2 | Gebläse des Beatmungsgeräts 100, umfasst den Einlass E, ist über einen Versorgungs-Ausgang mit dem ersten Kanal K.1 verbunden, fungiert in einer Ausgestaltung als die erste Quelle |
| 3 | signalverarbeitendes Steuergerät, empfängt Signale von den Sensoren 6.1, 6.2, 7.1, 7.2 und steuert die Proportionalventile 4.1 und 4.2 und in einer Ausgestaltung das Steuerdruck-Stellglied 17 an |
| 4.1 | Proportionalventil im ersten Kanal K.1, vermag den Volumenflusses durch den ersten Kanal K.1 zu verändern, wird vom Steuergerät 3 angesteuert |
| 4.2 | Proportionalventil im zweiten Kanal K.2, vermag den Volumenflusses durch den zweiten Kanal K.2 zu verändern, wird vom Steuergerät 3 angesteuert |
| 5.1 | pneumatischer Widerstand im ersten Kanal K.1 |
| 5.2 | pneumatischer Widerstand im zweiten Kanal K.2 |
| 6.1 | Volumenfluss-Sensor, misst die Druckdifferenz ΔP flussaufwärts und flussabwärts vom pneumatischen Widerstand 5.1 und leitet den Volumenfluss durch den ersten Kanal K.1 ab |
| 6.2 | Volumenfluss-Sensor, misst die Druckdifferenz ΔP flussaufwärts und flussabwärts vom pneumatischen Widerstand 5.2 und leitet den Volumenfluss durch den zweiten Kanal K.2 ab |
| 7.1 | Drucksensor, misst am Messpunkt 28.1 ein Maß für den Druck im ersten Kanal K.1 |
| 7.2 | Drucksensor, misst am Messpunkt 28.2 ein Maß für den Druck im zweiten Kanal K.2 |
| 8 | Mischpunkt, in den die beiden Kanäle K.1 und K.2 münden und in dem der Inspirations-Kanal K.30 beginnt |
| 9 | patientenseitige Koppeleinheit, über den Inspirations-Kanal K.30 mit dem Mischpunkt 8 verbunden |
| 10 | pneumatische Steuerleitung, führt vom Verzweigungspunkt 11 zur Steuerdruck-Kammer Ka.3 im Druckminderer 1, gehört zum Druckminderungs-Steuergerät |
| 11 | Verzweigungspunkt im ersten Kanal K.1, in dem die Steuerleitung 10 beginnt, beginnt, fungiert in einer Ausgestaltung als der Referenzpunkt |
| 12 | bewegliche Wand in Form einer Membrane im Druckminderer 1, trennt die Kammern Ka.2 und Ka.3 fluiddicht voneinander |
| 13 | Verschluss, der die Verbindung in der Trennwand 15 zwischen den beiden Kammern Ka.1 und Ka.2 wahlweise öffnet oder verschließt, steht über den Hebel 14 in einer Wirkverbindung mit der beweglichen Wand 12 |
| 14 | Hebel, stellt eine mechanische Wirkverbindung zwischen der beweglichen Wand 12 und den Verschluss 13 her, ist um die Drehachse DA drehbar |
| 15 | starre Trennwand im Druckminderer 1, welche die beiden Kammern Ka.1 und Ka.2 voneinander trennt, hat eine Öffnung 29, die durch den Verschluss 13 verschließbar ist |
| 16 | Feder, ist mit der beweglichen Wand 12 und mit dem Verbindungselement 18 verbunden, gehört zum Druckminderer-Stellglied |
| 17 | Steuerdruck-Stellglied, welches das Verbindungselement 18 bewegt und damit die Kraft der Feder 16 verändert, wird vom Steuergerät 3 angesteuert, gehört zum Druckminderer-Stellglied |
| 18 | mechanisches Verbindungselement zwischen dem Steuerdruck-Stellglied 17 und der Feder 16 |
| 19 | starres Gehäuse des Druckminderers 1, umschließt die drei Kammern Ka.1, Ka.2, Ka.3 |
| 20 | Versorgungsanschluss in der Wand W für reinen Sauerstoff, fungiert als die zweite Quelle |
| 21 | Versorgungsleitung, führt vom Versorgungsanschluss 20 zum Vordruck-Eingang V.3, verbindet den Versorgungsanschluss 20 mit der Vordruck-Kammer Ka.1 im Druckminderer 1 |
| 23 | Filter hinter dem Einlass E |
| 25 | CO2-Absorber, fungiert in einer Ausgestaltung als die erste Quelle |
| 26 | Rückschlagventil in der Versorgungsleitung 21 |
| 27 | Narkosemittelverdampfer, erzeugt gasförmiges Narkosemittel und speist dieses in den ersten Kanal K.1 ein |
| 28.1 | Messpunkt im ersten Kanal K.1, an dem der Drucksensor 7.1 den Druck im ersten Kanal K.1 misst, fungiert in einer Ausgestaltung als der Referenzpunkt |
| 28.2 | Messpunkt im zweiten Kanal K.2, an dem der Drucksensor 7.2 den Druck im zweiten Kanal K.2 misst |
| 29 | Öffnung in der Trennwand 15, wird vom Verschluss 13 wahlweise freigegeben oder verschlossen |
| 30 | Drehknopf, den ein Benutzer drehen kann, um den geforderten Anteil von Sauerstoff in dem Gasgemisch, das zur patientenseitigen Koppeleinheit 9 gefördert wird, vorzugeben |
| 31 | erster Abschnitt der Exspirations-Fluidverbindung, leitet die ausgeatmete Luft von der patientenseitigen Koppeleinheit 9 zum CO2-Absorber 25 |
| 32 | zweiter Abschnitt der Exspirations-Fluidverbindung, leitet die ausgeatmete und von CO2 befreite Luft vom CO2-Absorber 25 zum Gebläse 2 |
| 100 | Beatmungsgerät, erzeugt ein Gasgemisch aus Atemluft und reinem Sauerstoff, beatmet den Patienten Pt künstlich, umfasst die erfindungsgemäße Versorgungs-Anordnung |
| DA | Drehachse, um welche der Hebel 14 drehbar ist |
| E | Einlass für Umgebungsluft |
| K.1 | erster Kanal, stellt Atemluft bereit, steht in einer Fluidverbindung mit dem Gebläse 2, mündet in den Mischpunkt 8 |
| K.2 | zweiter Kanal, stellt reinen Sauerstoff bereit, steht in einer Fluidverbindung mit der Hinterdruck-Kammer Ka.2 im Druckminderer 1, mündet in den Mischpunkt 8 |
| K.30 | Inspirations-Kanal, leitet das Gasgemisch vom Mischpunkt 8 zur patientenseitigen Koppeleinheit 9 |
| Ka.1 | Vordruck-Kammer im Druckminderer 1, über den Vordruck-Eingang V.3 mit der Versorgungsleitung 21 verbunden |
| Ka.2 | Hinterdruck-Kammer im Druckminderer 1, über den Hinterdruck-Ausgang V.2 mit dem zweiten Kanal K.2 verbunden |
| Ka.3 | Steuerdruck-Kammer im Druckminderer 1, in einer Ausgestaltung über den Steuerdruck-Eingang V.4 mit der Steuerleitung 10 verbunden, enthält in einer anderen Ausgestaltung die Feder 16 und das Verbindungselement 18 |
| P | Druck, insbesondere im Inspirations-Kanal K.30 |
| P.1 | zeitlicher Verlauf des Drucks am Hinterdruck-Ausgang V.2 |
| P.2 | zeitlicher Verlauf des Drucks am Versorgungs-Verbindungselement V.1 |
| P.2(30) | zeitlicher Verlauf bei einem Solldruck von 30 mbar |
| P.2(50) | zeitlicher Verlauf bei einem Solldruck von 50 mbar |
| Pt | Patient, wird vom Beatmungsgerät 100 künstlich beatmet, trägt die patientenseitige Koppeleinheit 9 |
| V.1 | Versorgungs-Verbindungselement des ersten Kanals K.1, mit einem Versorgungs-Ausgang des Gebläses 2 verbunden |
| V.2 | Hinterdruck-Ausgang des Druckminderers 1, mit dem zweiten Kanal K.2 verbunden |
| V.3 | Vordruck-Eingang des Druckminderers 1, mit der Versorgungsleitung 21 verbunden |
| V.4 | optionaler Steuerdruck-Eingang des Druckminderers 1, mit der Steuerleitung 10 verbunden |
| W | Wand, hat den Versorgungsanschluss 20 für reinen Sauerstoff |

## Patentansprüche

1. Versorgungs-Anordnung zur Versorgung einer patientenseitigen Koppeleinheit (9)
mit einem Gasgemisch umfassend einen ersten Gas-Bestandteil (Luft) und einen zweiten Gas-Bestandteil (O2),
wobei die patientenseitige Koppeleinheit (9) mit einem Patienten (Pt) verbindbar oder wenigstens zeitweise verbunden ist,
wobei die Versorgungs-Anordnung
- einen ersten Kanal (K.1) mit einem Versorgungs-Verbindungselement (V.1),
- einen zweiten Kanal (K.2),
- einen Mischpunkt (8),
- einen Inspirations-Kanal (K.30) und
- einen Druckminderer (1) mit einem Vordruck-Eingang (V.3) und einem Hinterdruck-Ausgang (V.2)
umfasst,
wobei eine Fluidverbindung zwischen dem Versorgungs-Verbindungselement (V.1) und einer ersten Quelle (2, 25), nämlich einer Quelle für den ersten Gas-Bestandteil (Luft), hergestellt oder wenigstens zeitweise herstellbar ist,
wobei eine Fluidverbindung (21) zwischen dem Vordruck-Eingang (V.3) des Druckminderers (1) und einer zweiten Quelle (20), nämlich einer Quelle für den zweiten Gas-Bestandteil (O2), hergestellt oder wenigstens zeitweise herstellbar ist,
wobei der Hinterdruck-Ausgang (V.2) des Druckminderers (1) mit dem zweiten Kanal (K.2) verbunden ist,
wobei der erste Kanal (K.1) dazu ausgestaltet ist, den ersten Gas-Bestandteil (Luft) vom Versorgungs-Verbindungselement (V.1) zum Mischpunkt (8) zu leiten,
wobei der zweite Kanal (K.2) dazu ausgestaltet ist, den zweiten Gas-Bestandteil (O2) vom Hinterdruck-Ausgang (V.2) des Druckminderers (1) zum Mischpunkt (8) zu leiten,
wobei der Druckminderer (1) dazu ausgestaltet ist, den zweiten Gas-Bestandteil (O2) dergestalt bereitzustellen,
dass der zeitliche Verlauf (P.1) des Drucks am Hinterdruck-Ausgang (V.2) des Druckminderers (1) dem zeitlichen Verlauf (P.2) des Drucks an einem Referenzpunkt (11, 28.1) im ersten Kanal (K.1) folgt, und
wobei der Inspirations-Kanal (K.30) dazu ausgestaltet ist, ein im Mischpunkt (8) erzeugtes oder entstehendes Gasgemisch zur patientenseitigen Koppeleinheit (9) zu leiten,
**dadurch gekennzeichnet, dass**
die Versorgungs-Anordnung
- ein erstes Ventil (4.1),
- einen ersten Sensor (6.1, 7.1) sowie
- ein signalverarbeitendes Steuergerät (3)
umfasst,
wobei das erste Ventil (4.1) dazu ausgestaltet ist, den Volumenfluss (Vol') durch den ersten Kanal (K.1) und / oder den Druck (P) im ersten Kanal (K.1) zu verändern,
wobei das Steuergerät (3) dazu ausgestaltet ist,
- eine erste Regelung durchzuführen und
- bei der ersten Regelung das erste Ventil (4.1) abhängig von Messwerten des ersten Sensors (6.1, 7.1) anzusteuern,
wobei
- der erste Sensor (7.1) dazu ausgestaltet ist, ein Maß für den Druck (P) im ersten Kanal (K.1) zu messen und
- das Regelungsziel bei der ersten Regelung ist, dass der zeitliche Verlauf des tatsächlichen Drucks (P) im ersten Kanal (K.1) einem vorgegebenen Sollverlauf des Drucks folgt,
oder
- der erste Sensor (6.1) dazu ausgestaltet ist, ein Maß für den Volumenfluss (Vol') durch den ersten Kanal (K.1) zu messen und
- das Regelungsziel bei der ersten Regelung ist, dass der zeitliche Verlauf des tatsächlichen Volumenflusses (Vol') durch den ersten Kanal (K.1) einem vorgegebenen Sollverlauf des Volumenflusses folgt.

2. Versorgungs-Anordnung nach Anspruch 1,
**dadurch gekennzeichnet, dass**
die Versorgungs-Anordnung zusätzlich
- ein zweites Ventil (4.2) und
- einen zweiten Sensor (6.2, 7.2)
umfasst,
wobei das zweite Ventil (4.2) dazu ausgestaltet ist, den Volumenfluss (Vol') durch den zweiten Kanal (K.2) und / oder den Druck (P) im zweiten Kanal (K.2) zu verändern,
wobei das Steuergerät (3) dazu ausgestaltet ist,
- eine zweite Regelung durchzuführen und
- bei der zweiten Regelung das zweite Ventil (4.2) abhängig von Messwerten des zweiten Sensors (6.2, 7.2) anzusteuern,
wobei
- der zweite Sensor (7.2) dazu ausgestaltet ist, ein Maß für den Druck (P) im zweiten Kanal (K.2) zu messen und
- das Regelungsziel bei der zweiten Regelung ist, dass der zeitliche Verlauf des tatsächlichen Drucks (P) im zweiten Kanal (K.2) einem vorgegebenen Sollverlauf des Drucks folgt,
oder
- der zweite Sensor (6.2) dazu ausgestaltet ist, ein Maß für den Volumenfluss (Vol') durch den zweiten Kanal (K.2) zu messen und
- das Regelungsziel bei der zweiten Regelung ist, dass der zeitliche Verlauf des tatsächlichen Volumenflusses (Vol') durch den zweiten Kanal (K.2) einem vorgegebenen Sollverlauf des Volumenflusses folgt.

3. Versorgungs-Anordnung nach einem der vorhergehenden Ansprüche, **dadurch gekennzeichnet, dass**
die Versorgungs-Anordnung eine pneumatische Steuerleitung (10) umfasst, wobei die pneumatische Steuerleitung (10)
- mit dem Druckminderer (1) und
- in einem Abzweigungspunkt (11) mit dem ersten Kanal (K.1) pneumatisch verbunden ist,
wobei die pneumatische Steuerleitung (10) dergestalt eine Fluidverbindung zwischen dem ersten Kanal (K.1) und dem Druckminderer (1) herstellt, dass der zeitlichen Verlauf des Drucks in der pneumatischen Steuerleitung (10) dem zeitlichen Verlauf (P.2) des Drucks im Abzweigungspunkt (11) folgt, und
wobei der Druckminderer (1) dazu ausgestaltet ist zu bewirken,
dass der zeitliche Verlauf (P.1) des Drucks am Hinterdruck-Ausgang (V.2) des Druckminderers (1) dem zeitlichen Verlauf des Drucks in der pneumatischen Steuerleitung (10) folgt.

4. Versorgungs-Anordnung nach Anspruch 3,
**dadurch gekennzeichnet, dass**
im Inneren des Druckminderers (1)
- eine Vordruck-Kammer (Ka.1), die über den Vordruck-Eingang (V.3) des Druckminderers (1) mit der ersten Quelle (20) verbindbar oder wenigstens zeitweise verbunden ist,
- eine Hinterdruck-Kammer (Ka.2), die über den Hinterdruck-Ausgang (V.2) des Druckminderers (1) mit dem zweiten Kanal (K.2) verbunden ist, und
- eine Steuerdruck-Kammer (Ka.3)
ausgebildet sind,
wobei die pneumatische Steuerleitung (10) dergestalt in einer Fluidverbindung mit der Steuerdruck-Kammer (Ka.3) steht, dass der Druck in der Steuerdruck-Kammer (Ka.3) dem Druck in der pneumatischen Steuerleitung (10) folgt, und
wobei der Druckminderer (1) so ausgebildet ist, dass der zeitliche Verlauf (P.1) des Drucks am Hinterdruck-Ausgang (V.2) dem zeitlichen Verlauf (P.2) des Drucks in der Steuerdruck-Kammer (Ka.3) folgt.

5. Versorgungs-Anordnung nach Anspruch 4,
**dadurch gekennzeichnet, dass**
der Druckminderer (1)
- eine Trennwand (15) mit einer Öffnung (29) und
- einen Verschluss (13)
umfasst,
wobei die Trennwand (15) die Vordruck-Kammer (Ka.1) von der Hinterdruck-Kammer (Ka.2) trennt,
wobei die Öffnung (29) in der Trennwand (15) die Vordruck-Kammer (Ka.1) mit der Hinterdruck-Kammer (Ka.2) verbindet,
wobei der Verschluss (13) die Öffnung (29) in der Trennwand (15) wahlweise freizugeben oder zu verschließen vermag und
wobei der Druckminderer (1) so ausgestaltet ist, dass der Verschluss (13)
- die Öffnung (29) dann freigibt, wenn ein vorgegebenes Kriterium erfüllt ist,
wobei das Kriterium vom Druck in der Steuerdruck-Kammer (Ka.3) und / oder vom Druck in der Hinterdruck-Kammer (Ka.2) abhängt, und
- ansonsten die Öffnung (29) verschließt.

6. Versorgungs-Anordnung nach Anspruch 5,
**dadurch gekennzeichnet, dass**
der Druckminderer (1) ein Gehäuse (19) umfasst und
eine Wand (12) die Hinterdruck-Kammer (Ka.2) von der Steuerdruck-Kammer (Ka.3) trennt,
wobei die Wand (12) zwischen der Hinterdruck-Kammer (Ka.2) und der Steuerdruck-Kammer (Ka.3) dergestalt relativ zum Gehäuse (19) des Druckminderers (1) beweglich oder flexibel ist,
dass das Volumen der Hinterdruck-Kammer (Ka.2) und das Volumen der Steuerdruck-Kammer (Ka.3) veränderbar sind,
wobei die bewegliche oder flexible Wand (12) in einer Wirkverbindung (14) mit dem Verschluss (13) für die Öffnung (29) in der Trennwand (15) steht.

7. Versorgungs-Anordnung nach einem der vorhergehenden Ansprüche, **dadurch gekennzeichnet, dass**
die Versorgungs-Anordnung einen Drucksensor (7.1) und ein signalverarbeitendes Druckminderungs-Steuergerät (3) umfasst,
wobei der Drucksensor (7.1) dazu ausgestaltet ist, ein Maß für den Druck an einem Messpunkt (28.1) im ersten Kanal (K.1) zu messen, und
wobei das Druckminderungs-Steuergerät (3) dazu ausgestaltet ist, abhängig von einen Signal des Drucksensors (7.1) zu bewirken, dass der zeitliche Verlauf (P.1) des Drucks am Hinterdruck-Ausgang (V.2) dem zeitlichen Verlauf (P.2) des Drucks am Referenzpunkt (28.1) im ersten Kanal (K.1) folgt,
wobei bevorzugt der Messpunkt (28.1) gleich dem Referenzpunkt (28.1) ist.

8. Versorgungs-Anordnung nach Anspruch 7,
**dadurch gekennzeichnet, dass**
im Inneren des Druckminderers (1) eine Hinterdruck-Kammer (Ka.2), die über den Hinterdruck-Ausgang (V.2) des Druckminderers (1) mit dem zweiten Kanal (K.2) verbunden ist, ausgebildet ist und
der Druckminderer (1) ein ansteuerbares Druckminderer-Stellglied (16, 17, 18) umfasst,
wobei das Druckminderungs-Steuergerät (3) dazu ausgestaltet ist, abhängig von einem Signal des Drucksensors (7.1)
- das Druckminderer-Stellglied (16, 17, 18) anzusteuern und
- durch die Ansteuerung zu bewirken, dass der zeitliche Verlauf (P.1) des Drucks in der Hinterdruck-Kammer (Ka.2) dem zeitlichen Verlauf (P.2) des Drucks am Referenzpunkt (28.1) im ersten Kanal (K.1) folgt.

9. Versorgungs-Anordnung nach Anspruch 8,
**dadurch gekennzeichnet, dass**
der Druckminderer (1)
- eine Vordruck-Kammer (Ka.1), die über den Vordruck-Eingang (V.3) mit der ersten Quelle (20) verbindbar oder wenigstens zeitweise verbunden ist,
- eine Trennwand (15) mit einer Öffnung (29) und
- einen Verschluss (13)
umfasst,
wobei die Trennwand (15) die Vordruck-Kammer (Ka.1) von der Hinterdruck-Kammer (Ka.2) trennt,
wobei die Öffnung (29) in der Trennwand (15) die Vordruck-Kammer (Ka.1) mit der Hinterdruck-Kammer (Ka.2) verbindet,
wobei der Verschluss (13) die Öffnung (29) in der Trennwand (15) wahlweise freizugeben oder zu verschließen vermag und
wobei das Druckminderer-Stellglied (16, 17, 18) in einer Wirkverbindung (14) mit dem Verschluss (13) steht.

10. Versorgungs-Anordnung nach Anspruch 8 oder Anspruch 9,
**dadurch gekennzeichnet, dass**
eine Wand (12) der Hinterdruck-Kammer (Ka.2) dergestalt relativ zu einer weiteren Wand (19) der Hinterdruck-Kammer (Ka.2) beweglich ist, dass das Volumen der Hinterdruck-Kammer (Ka.2) veränderbar ist,
wobei das Druckminderer-Stellglied (16, 17, 18) in einer Wirkverbindung (14) mit der beweglichen Wand (12) steht.

11. Versorgungs-System zur Versorgung einer patientenseitigen Koppeleinheit (9)
mit einem Gasgemisch umfassend einen ersten Gas-Bestandteil (Luft) und einen zweiten Gas-Bestandteil (O2),
wobei das Versorgungs-System
- eine erste Quelle (2, 25),
- eine zweite Quelle (20) und
- eine Versorgungs-Anordnung nach einem der vorhergehenden Ansprüche
umfasst,
wobei die erste Quelle (2, 25) dazu ausgestaltet ist, den ersten Gas-Bestandteil (Luft) bereitzustellen,
wobei die zweite Quelle (20) dazu ausgestaltet ist, den zweiten Gas-Bestandteil (O2) bereitzustellen,
wobei eine Fluidverbindung zwischen dem Versorgungs-Verbindungselement (V.1) der Versorgungs-Anordnung und der ersten Quelle (2, 25) herstellbar oder wenigstens zeitweise hergestellt ist und
wobei eine Fluidverbindung (21) zwischen dem Vordruck-Eingang (V.3) des Druckminderers (1) und der zweiten Quelle (20) (20) herstellbar oder wenigstens zweitweise hergestellt ist.

12. Versorgungs-System nach Anspruch 11,
**dadurch gekennzeichnet, dass**
die zweite Quelle (20) den zweiten Gas-Bestandteil (O2) mit einem höheren Druck bereitstellt als die erste Quelle (2) den ersten Gas-Bestandteil (Luft).

13. Beatmungs-System (100) zur künstlichen Beatmung eines Patienten (Pt) mit einem Gasgemisch umfassend einen ersten Gas-Bestandteil (Luft) und einen zweiten Gas-Bestandteil (O2),
wobei mindestens einer der beiden Gas-Bestandteile (Luft, O2) Sauerstoff ist oder Sauerstoff enthält,
wobei das Beatmungs-System
- eine Fluidförderereinheit (2),
- eine patientenseitige Koppeleinheit (9) und
- eine Versorgungs-Anordnung nach einem der Ansprüche 1 bis 10 oder ein Versorgungs-System nach Anspruch 11 oder Anspruch 12
umfasst,
wobei die patientenseitige Koppeleinheit (9) mit einem Patienten (Pt) verbindbar oder wenigstens zeitweise verbunden ist,
wobei eine Fluidverbindung zwischen dem Versorgungs-Verbindungselement (V.1) der Versorgungs-Anordnung und einer ersten Quelle (2, 25) herstellbar oder wenigstens zeitweise hergestellt ist, ist, nämlich einer Quelle für den ersten Gas-Bestandteil (Luft),
wobei die Fluidführungseinheit (2) dazu ausgestaltet ist, den ersten Gas-Bestandteil (Luft) von der ersten Quelle (2, 25) durch den ersten Kanal (K.1) hindurch zum Mischpunkt (8) zu fördern,
wobei eine Fluidverbindung (21) zwischen dem Vordruck-Eingang (V.3) des Druckminderers (1) und einer zweiten Quelle (20) hergestellt oder wenigstens zeitweise herstellbar ist, nämlich einer Quelle für den zweiten Gas-Bestandteil (O2), und
wobei das Beatmungs-System (100) dazu ausgestaltet ist,
- Beatmungshübe durchzuführen und
- in jedem Beatmungshub jeweils eine Menge des Gasgemischs durch den Inspirations-Kanal (K.30) hindurch zur patientenseitigen Koppeleinheit (9) zu leiten.

14. Beatmungs-System (100) nach Anspruch 14,
**dadurch gekennzeichnet, dass**
das erste Ventil (4.1) dazu ausgestaltet ist, den Volumenfluss durch den ersten Kanal (K.1) zu verändern,
wobei das Steuergerät (3) dazu ausgestaltet ist,
- abhängig von einem vorgegebenen zeitlichen Soll-Verlauf des Volumenflusses (Vol') des Gasgemisches einen Soll-Verlauf des Volumenflusses des ersten Gas-Bestandteils (Luft) herzuleiten und
- das erste Ventil (4.1) mit dem Regelungsziel anzusteuern, dass der tatsächliche Volumenfluss durch den ersten Kanal (K.1) dem hergeleiteten Soll-Verlauf des Volumenflusses des ersten Gas-Bestandteils (Luft) gleicht.

15. Beatmungs-System (100) nach Anspruch 13 oder Anspruch 14,
**dadurch gekennzeichnet, dass**
das Beatmungs-System (100) ein zweites Ventil (4.2) umfasst,
wobei das zweite Ventil (4.2) dazu ausgestaltet ist, den Volumenfluss durch den zweiten Kanal (K.2) zu verändern, und
wobei das Steuergerät (3) dazu ausgestaltet ist,
- abhängig von einem vorgegebenen zeitlichen Soll-Verlauf des Volumenflusses (Vol') des Gasgemisches einen Soll-Verlauf des Volumenflusses des zweiten Gas-Bestandteils (O2) herzuleiten und
- das zweite Ventil (4.2) mit dem Regelungsziel anzusteuern, dass der tatsächliche Volumenfluss durch den zweiten Kanal (K.2) dem hergeleiteten Soll-Verlauf des Volumenflusses des zweiten Gas-Bestandteils (O2) gleicht.

## Claims

1. Supply arrangement for supplying a patient-side coupling unit (9) with a gas mixture comprising a first gas component (air) and a second gas component (O2),
the patient-side coupling unit (9) being connectable or at least temporarily connected to a patient (Pt),
the supply arrangement comprising
- a first duct (K.1) having a supply connection element (V.1),
- a second duct (K.2),
- a mixing point (8),
- an inspiratory duct (K.30) and
- a pressure regulator (1) having an upstream pressure inlet (V.3) and a downstream pressure outlet (V.2),
a fluid connection being established or at least temporarily establishable between the supply connection element (V. 1) and a first source (2, 25), namely a source for the first gas component (air),,
a fluid connection (21) being established or at least temporarily establishable between the upstream pressure inlet (V.3) of the pressure regulator (1) and a second source (20), namely a source for the second gas component (O2),
the downstream pressure outlet (V.2) of the pressure regulator (1) being connected to the second duct (K.2),
the first duct (K.1) being designed to guide the first gas component (air) from the supply connection element (V.1) to the mixing point (8),
the second duct (K.2) being designed to guide the second gas component (O2) from the downstream pressure outlet (V.2) of the pressure regulator (1) to the mixing point (8),
the pressure regulator (1) being designed to provide the second gas component (O2) in such a way
that the time course (P.1) of the pressure at the downstream pressure outlet (V.2) of the pressure regulator (1) follows the time course (P.2) of the pressure at a reference point (11, 28.1) in the first duct (K.1), and
the inspiratory duct (K.30) being designed to conduct a gas mixture generated or created in the mixing point (8) to the patient-side coupling unit (9),
**characterized in that**
the supply arrangement comprises
- a first valve (4.1),
- a first sensor (6.1, 7.1) and
- a signal processing controller (3),
the first valve (4.1) being designed to change the volume flow (Vol') through the first duct (K.1) and/or the pressure (P) in the first duct (K.1),
the controller (3) being designed
- to carry out a first closed-loop control and
- during the first closed-loop control, to control the first valve (4.1) depending on the measured values of the first sensor (6.1, 7.1),
- the first sensor (7.1) being designed to measure a measure of the pressure (P) in the first duct (K.1) and
- the control objective in the first closed-loop control being that the time course of the actual pressure (P) in the first duct (K.1) follows a predetermined target course of the pressure,
or
- the first sensor (6.1) being designed to measure a measure of the volume flow (Vol') through the first duct (K.1) and
- the control objective in the first closed-loop control being that the time course of the actual volume flow (Vol') through the first duct (K.1) follows a predetermined target course of the volume flow.

2. Supply arrangement according to claim 1,
**characterized in that**
the supply arrangement additionally comprises
- a second valve (4.2) and
- a second sensor (6.2, 7.2),
the second valve (4.2) being designed to change the volume flow (Vol') through the second duct (K.2) and/or the pressure (P) in the second duct (K.2),
the controller (3) being designed
- to carry out a second closed-loop control and
- during the second closed-loop control, to control the second valve (4.2) depending on the measured values of the second sensor (6.2, 7.2),
- the second sensor (7.2) being designed to measure a measure of the pressure (P) in the second duct (K.2) and
- the control objective in the second closed-loop control being that the time course of the actual pressure (P) in the second duct (K.2) follows a predetermined target course of the pressure,
or
- the second sensor (6.2) being designed to measure a measure of the volume flow (Vol') through the second duct (K.2) and
- the control objective of the second closed-loop control being that the time course of the actual volume flow (Vol') through the second duct (K.2) follows a predetermined target course of the volume flow.

3. Supply arrangement according to either of the preceding claims, **characterized in that**
the supply arrangement comprises a pneumatic control line (10),
the pneumatic control line (10) being pneumatically connected
- to the pressure regulator (1) and
- to the first duct (K. 1) at a branch-off point (11),
the pneumatic control line (10) establishing a fluid connection between the first duct (K.1) and the pressure regulator (1) in such a way that the time course of the pressure in the pneumatic control line (10) follows the time course (P.2) of the pressure at the branch-off point (11), and
the pressure regulator (1) being designed to cause
the time course (P.1) of the pressure at the downstream pressure outlet (V.2) of the pressure regulator (1) to follow the time course of the pressure in the pneumatic control line (10).

4. Supply arrangement according to claim 3,
**characterized in that**
inside the pressure regulator (1)
- an upstream pressure chamber (Ka. 1), which is connectable or at least temporarily connected to the first source (20) via the upstream pressure inlet (V.3) of the pressure regulator (1),
- a downstream pressure chamber (Ka.2), which is connected to the second duct (K.2) via the downstream pressure outlet (V.2) of the pressure regulator (1), and
- a control pressure chamber (Ka.3)
are formed,
the pneumatic control line (10) being in fluid connection with the control pressure chamber (Ka.3) in such a way that the pressure in the control pressure chamber (Ka.3) follows the pressure in the pneumatic control line (10), and
the pressure regulator (1) being designed such that the time course (P.1) of the pressure at the downstream pressure outlet (V.2) follows the time course (P.2) of the pressure in the control pressure chamber (Ka.3).

5. Supply arrangement according to claim 4,
**characterized in that**
the pressure regulator (1) comprises
- a partition (15) having an opening (29) and
- a closure (13),
the partition (15) separating the upstream pressure chamber (Ka.1) from the downstream pressure chamber (Ka.2),
the opening (29) in the partition (15) connecting the upstream pressure chamber (Ka.1) to the downstream pressure chamber (Ka.2),
the closure (13) being capable of selectively opening or closing the opening (29) in the partition (15) and
the pressure regulator (1) being designed such that the closure (13)
- opens the opening (29) when a predetermined criterion is met,
the criterion depending on the pressure in the control pressure chamber (Ka.3) and/or the pressure in the downstream pressure chamber (Ka.2), and
- otherwise closes the opening (29).

6. Supply arrangement according to claim 5,
**characterized in that**
the pressure regulator (1) comprises a housing (19) and
a wall (12) separates the downstream pressure chamber (Ka.2) from the control pressure chamber (Ka.3),
the wall (12) between the downstream pressure chamber (Ka.2) and the control pressure chamber (Ka.3) being movable or flexible relative to the housing (19) of the pressure regulator (1) in such a way
that the volume of the downstream pressure chamber (Ka.2) and the volume of the control pressure chamber (Ka.3) are changeable,
the movable or flexible wall (12) being in operative connection (14) with the closure (13) for the opening (29) in the partition (15).

7. Supply arrangement according to any of the preceding claims, **characterized in that**
the supply arrangement comprises a pressure sensor (7.1) and a signalprocessing pressure regulation controller (3),
the pressure sensor (7.1) being designed to measure a measure of the pressure at a measuring point (28.1) in the first duct (K.1), and
the pressure regulation controller (3) being designed,
depending on a signal from the pressure sensor (7.1), to cause the time course (P.1) of the pressure at the downstream pressure outlet (V.2) to follow the time course (P.2) of the pressure at the reference point (28.1) in the first duct (K.1),
the measuring point (28.1) preferably being equal to the reference point (28.1).

8. Supply arrangement according to claim 7,
**characterized in that**
a downstream pressure chamber (Ka.2) that is connected to the second duct (K.2) via the downstream pressure outlet (V.2) of the pressure regulator (1) is formed inside the pressure regulator (1) and
the pressure regulator (1) comprises a controllable pressure regulator actuator (16, 17, 18),
the pressure regulation controller (3) being designed,
depending on a signal from the pressure sensor (7.1),
- to control the pressure regulator actuator (16, 17, 18) and
- to cause, through the control, the time course (P.1) of the pressure in the downstream pressure chamber (Ka.2) to follow the time course (P.2) of the pressure at the reference point (28.1) in the first duct (K.1).

9. Supply arrangement according to claim 8,
**characterized in that**
the pressure regulator (1) comprises
- an upstream pressure chamber (Ka.1) which is connectable or is at least temporarily connected to the first source (20) via the upstream pressure inlet (V.3),
- a partition (15) having an opening (29) and
- a closure (13),
the partition (15) separating the upstream pressure chamber (Ka.1) from the downstream pressure chamber (Ka.2),
the opening (29) in the partition (15) connecting the upstream pressure chamber (Ka.1) to the downstream pressure chamber (Ka.2),
the closure (13) being capable of selectively opening or closing the opening (29) in the partition (15) and
the pressure regulator actuator (16, 17, 18) being in operative connection (14) with the closure (13).

10. Supply arrangement according to either claim 8 or claim 9, **characterized in that**
a wall (12) of the downstream pressure chamber (Ka.2) is movable relative to another wall (19) of the downstream pressure chamber (Ka.2) in such a way that the volume of the downstream pressure chamber (Ka.2) is changeable,
the pressure regulator actuator (16, 17, 18) being in operative connection (14) with the movable wall (12).

11. Supply system for supplying a patient-side coupling unit (9) with a gas mixture comprising a first gas component (air) and a second gas component (O2),
wherein the supply system comprises
- a first source (2, 25),
- a second source (20) and
- a supply arrangement according to any of the preceding claims,
wherein the first source (2, 25) is designed to provide the first gas component (air),
wherein the second source (20) is designed to provide the second gas component (O2),
wherein a fluid connection is establishable or is at least temporarily established between the supply connection element (V.1) of the supply arrangement and the first source (2, 25) and
wherein a fluid connection (21) is establishable or is at least temporarily established between the upstream pressure inlet (V.3) of the pressure regulator (1) and the second source (20) (20).

12. Supply system according to claim 11,
**characterized in that**
the second source (20) provides the second gas component (O2) at a higher pressure than the first source (2) provides the first gas component (air).

13. Ventilation system (100) for artificial ventilation of a patient (Pt) with a gas mixture comprising a first gas component (air) and a second gas component (O2),
wherein at least one of the two gas components (air, O2) is oxygen or contains oxygen,
wherein the ventilation system comprises
- a fluid delivery unit (2),
- a patient-side coupling unit (9) and
- a supply arrangement according to any of claims 1 to 10 or a supply system according to either claim 11 or claim 12,
the patient-side coupling unit (9) being connectable or at least temporarily connected to a patient (Pt),
wherein a fluid connection is establishable or is at least temporarily established between the supply connection element (V.1) of the supply arrangement and a first source (2, 25), namely a source for the first gas component (air),
wherein the fluid guide unit (2) is designed to deliver the first gas component (air) from the first source (2, 25) through the first duct (K.1) to the mixing point (8),
wherein a fluid connection (21) is established or is at least temporarily establishable between the upstream pressure inlet (V.3) of the pressure regulator (1) and a second source (20), namely a source for the second gas component (O2), and
wherein the ventilation system (100) is designed
- to carry out ventilation strokes and
- to direct in each ventilation stroke a quantity of the gas mixture through the inspiratory duct (K.30) to the patient-side coupling unit (9).

14. Ventilation system (100) according to claim 14,
**characterized in that**
the first valve (4.1) is designed to change the volume flow through the first duct (K.1),
the controller (3) being designed
- to derive a target course of the volume flow of the first gas component (air) depending on a predetermined target time course of the volume flow (Vol') of the gas mixture and
- to control the first valve (4.1) using the control objective such that the actual volume flow through the first duct (K.1) equals the derived target course of the volume flow of the first gas component (air).

15. Ventilation system (100) according to either claim 13 or claim 14, **characterized in that**
the ventilation system (100) comprises a second valve (4.2),
the second valve (4.2) being designed to change the volume flow through the second duct (K.2), and
the controller (3) being designed
- to derive a target course of the volume flow of the second gas component (O2) depending on a predetermined target time course of the volume flow (Vol') of the gas mixture and
- to control the second valve (4.2) using the control objective such that the actual volume flow through the second duct (K.2) equals the derived target course of the volume flow of the second gas component (O2).

## Revendications

1. Agencement d'alimentation pour l'alimentation d'une unité de couplage (9) côté patient avec un mélange de gaz comprenant un premier composant gazeux (air) et un second composant gazeux (O2),
dans lequel l'unité de couplage (9) côté patient peut être reliée ou est reliée au moins temporairement à un patient (Pt),
dans lequel l'agencement d'alimentation comprend
- un premier canal (K.1) comportant un élément de liaison d'alimentation (V.1),
- un second canal (K.2),
- un point de mélange (8),
- un canal d'inspiration (K.30) et
- un détendeur (1) comportant une entrée de pression amont (V.3) et une sortie de pression aval (V.2),
dans lequel une liaison fluidique est établie ou peut être établie au moins temporairement entre l'élément de liaison d'alimentation (V. 1) et une première source (2, 25), à savoir une source pour le premier composant gazeux (air),
dans lequel une liaison fluidique (21) est établie ou peut être établie au moins temporairement entre l'entrée de pression amont (V.3) du détendeur (1) et une seconde source (20), à savoir une source pour le second composant gazeux (O2),
dans lequel la sortie de pression aval (V.2) du détendeur (1) est reliée au second canal (K.2),
dans lequel le premier canal (K.1) est conçu pour conduire le premier composant gazeux (air) de l'élément de liaison d'alimentation (V.1) au point de mélange (8),
dans lequel le second canal (K.2) est conçu pour conduire le second composant gazeux (O2) de la sortie de pression arrière (V.2) du détendeur (1) au point de mélange (8),
dans lequel le détendeur (1) est conçu pour fournir le second composant gazeux (O2) de telle sorte,
que la courbe temporelle (P. 1) de la pression à la sortie de pression aval (V.2) du détendeur (1) suit la courbe temporelle (P.2) de la pression en un point de référence (11, 28.1) dans le premier canal (K.1), et
dans lequel le canal d'inspiration (K.30) est conçu pour conduire un mélange de gaz produit ou se formant au point de mélange (8) à l'unité de couplage (9) côté patient,
**caractérisé en ce que**
l'agencement d'alimentation comprend
- une première soupape (4.1),
- un premier capteur (6.1, 7.1) ainsi que
- un appareil de commande (3) traitant les signaux,
dans lequel la première soupape (4.1) est conçue pour modifier le débit volumique (Vol') à travers le premier canal (K.1) et/ou la pression (P) dans le premier canal (K.1),
dans lequel l'appareil de commande (3) est conçu pour
- mettre en œuvre une première régulation et
- lors de la première régulation, commander la première soupape (4.1) en fonction des valeurs de mesure du premier capteur (6.1, 7.1),
dans lequel
- le premier capteur (7.1) est conçu pour mesurer une mesure de la pression (P) dans le premier canal (K.1) et
- l'objectif de régulation lors de la première régulation est que la courbe temporelle de la pression (P) effective dans le premier canal (K.1) suive une courbe de consigne prédéfinie de la pression,
ou
- le premier capteur (6.1) est conçu pour mesurer une mesure du débit volumique (Vol') à travers le premier canal (K.1) et
- l'objectif de régulation lors de la première régulation est que la courbe temporelle du débit volumique (Vol') effectif à travers le premier canal (K.1) suive une courbe de consigne prédéfinie du débit volumique.

2. Agencement d'alimentation selon la revendication 1,
**caractérisé en ce que**
l'agencement d'alimentation comprend de plus
- une seconde soupape (4.2) et
- un second capteur (6.2, 7.2),
dans lequel la seconde soupape (4.2) est conçue pour modifier le débit volumique (Vol') à travers le second canal (K.2) et/ou la pression (P) dans le second canal (K.2),
dans lequel l'appareil de commande (3) est conçu pour
- mettre en œuvre une seconde régulation et
- lors de la seconde régulation, commander la seconde soupape (4.2) en fonction des valeurs de mesure du second capteur (6.2, 7.2),
dans lequel
- le second capteur (7.2) est conçu pour mesurer une mesure de la pression (P) dans le second canal (K.2) et
- l'objectif de régulation lors de la seconde régulation est que la courbe temporelle de la pression (P) effective dans le second canal (K.2) suive une courbe de consigne prédéfinie de la pression,
ou
- le second capteur (6.2) est conçu pour mesurer une mesure du débit volumique (Vol') à travers le second canal (K.2) et
- l'objectif de régulation lors de la seconde régulation est que la courbe temporelle du débit volumique (Vol') effectif à travers le second canal (K.2) suive une courbe de consigne prédéfinie du débit volumique.

3. Agencement d'alimentation selon l'une des revendications précédentes, **caractérisé en ce que**
l'agencement d'alimentation comprend une conduite de commande pneumatique (10),
dans lequel la conduite de commande pneumatique (10) est reliée
- au détendeur (1) et
- en un point de dérivation (11) au premier canal (K. 1)
par voie pneumatique,
dans lequel la conduite de commande pneumatique (10) établit une liaison fluidique entre le premier canal (K.1) et le détendeur (1) de telle sorte que la courbe temporelle de la pression dans la conduite de commande pneumatique (10) suit la courbe temporelle (P.2) de la pression dans le point de dérivation (11), et
dans lequel le détendeur (1) est conçu pour entraîner,
que la courbe temporelle (P.1) de la pression à la sortie de pression aval (V.2) du détendeur (1) suive la courbe temporelle de la pression dans la conduite de commande pneumatique (10).

4. Agencement d'alimentation selon la revendication 3,
**caractérisé en ce**
**qu'**à l'intérieur du détendeur (1)
- une chambre de pression amont (Ka. 1), qui peut être reliée ou est reliée au moins temporairement à la première source (20) par l'intermédiaire de l'entrée de pression amont (V.3) du détendeur (1),
- une chambre de pression aval (Ka.2) qui est reliée au second canal (K.2) par l'intermédiaire de la sortie de pression aval (V.2) du détendeur (1), et
- une chambre de pression de commande (Ka.3)
sont réalisées,
dans lequel la conduite de commande pneumatique (10) est en liaison fluidique avec la chambre de pression de commande (Ka.3) de telle sorte que la pression dans la chambre de pression de commande (Ka.3) suit la pression dans la conduite de commande pneumatique (10), et
dans lequel le détendeur (1) est réalisé de telle sorte que la courbe temporelle (P.1) de la pression à la sortie de pression aval (V.2) suit la courbe temporelle (P.2) de la pression dans la chambre de pression de commande (Ka.3).

5. Agencement d'alimentation selon la revendication 4,
**caractérisé en ce que**
le détendeur (1) comprend
- une paroi de séparation (15) comportant une ouverture (29) et
- un obturateur (13),
dans lequel la paroi de séparation (15) sépare la chambre de pression amont (Ka.1) de la chambre de pression aval (Ka.2),
dans lequel l'ouverture (29) dans la paroi de séparation (15) relie la chambre de pression amont (Ka.1) à la chambre de pression aval (Ka.2),
dans lequel l'obturateur (13) est apte à libérer ou à fermer sélectivement l'ouverture (29) dans la paroi de séparation (15), et
dans lequel le détendeur (1) est conçu de telle sorte que l'obturateur (13)
- libère l'ouverture (29) lorsqu'un critère prédéterminé est rempli,
dans lequel le critère dépend de la pression dans la chambre de pression de commande (Ka.3) et/ou de la pression dans la chambre de pression aval (Ka.2), et
- sinon, ferme l'ouverture (29).

6. Agencement d'alimentation selon la revendication 5,
**caractérisé en ce que**
le détendeur (1) comprend un boîtier (19) et
une paroi (12) sépare la chambre de pression aval (Ka.2) de la chambre de pression de commande (Ka.3),
dans lequel la paroi (12) entre la chambre de pression aval (Ka.2) et la chambre de pression de commande (Ka.3) est mobile ou flexible de telle sorte par rapport au boîtier (19) du détendeur (1),
que le volume de la chambre de pression aval (Ka.2) et le volume de la chambre de pression de commande (Ka.3) sont modifiables,
dans lequel la paroi mobile ou flexible (12) est en liaison fonctionnelle (14) avec l'obturateur (13) pour l'ouverture (29) dans la paroi de séparation (15).

7. Agencement d'alimentation selon l'une des revendications précédentes, **caractérisé en ce que**
l'agencement d'alimentation comprend un capteur de pression (7.1) et un appareil de commande de détente (3) traitant des signaux,
dans lequel le capteur de pression (7.1) est conçu pour mesurer une mesure de la pression en un point de mesure (28.1) dans le premier canal (K.1), et
dans lequel l'appareil de commande de détente (3) est conçu pour,
en fonction d'un signal du capteur de pression (7.1), entraîner que la courbe temporelle (P.1) de la pression à la sortie de pression aval (V.2) suive la courbe temporelle (P.2) de la pression au point de référence (28.1) dans le premier canal (K.1),
dans lequel le point de mesure (28.1) est de préférence égal au point de référence (28.1).

8. Agencement d'alimentation selon la revendication 7, **caractérisé en ce**
**qu'**à l'intérieur du détendeur (1) est réalisée une chambre de pression aval (Ka.2) qui est reliée au second canal (K.2) par l'intermédiaire de la sortie de pression aval (V.2) du détendeur (1), et
le détendeur (1) comprend un organe de réglage de détendeur (16, 17, 18) pouvant être commandé,
dans lequel l'appareil de commande de détente (3) est conçu pour,
en fonction d'un signal du capteur de pression (7.1)
- commander l'organe de réglage de détendeur (16, 17, 18) et
- entraîner, par la commande, que la courbe temporelle (P.1) de la pression dans la chambre de pression aval (Ka.2) suive la courbe temporelle (P.2) de la pression au point de référence (28.1) dans le premier canal (K.1).

9. Agencement d'alimentation selon la revendication 8,
**caractérisé en ce que**
le détendeur (1) comprend
- une chambre de pression amont (Ka.1) qui peut être reliée ou est reliée au moins temporairement à la première source (20) par l'intermédiaire de l'entrée de pression amont (V.3),
- une paroi de séparation (15) comportant une ouverture (29) et
- un obturateur (13),
dans lequel la paroi de séparation (15) sépare la chambre de pression amont (Ka.1) de la chambre de pression aval (Ka.2),
dans lequel l'ouverture (29) dans la paroi de séparation (15) relie la chambre de pression amont (Ka.1) à la chambre de pression aval (Ka.2),
dans lequel l'obturateur (13) est apte à libérer ou à fermer sélectivement l'ouverture (29) dans la paroi de séparation (15), et
dans lequel l'organe de réglage de détendeur (16, 17, 18) est en liaison fonctionnelle (14) avec l'obturateur (13).

10. Agencement d'alimentation selon la revendication 8 ou la revendication 9, **caractérisé en ce**
**qu'**une paroi (12) de la chambre de pression aval (Ka.2) est mobile par rapport à une autre paroi (19) de la chambre de pression aval (Ka.2) de telle sorte que le volume de la chambre de pression aval (Ka.2) est modifiable,
dans lequel l'organe de réglage de détendeur (16, 17, 18) est en liaison fonctionnelle (14) avec la paroi mobile (12).

11. Système d'alimentation pour l'alimentation d'une unité de couplage (9) côté patient avec un mélange de gaz comprenant un premier composant gazeux (air) et un second composant gazeux (O2),
dans lequel le système d'alimentation comprend
- une première source (2, 25),
- une seconde source (20) et
- un agencement d'alimentation selon l'une des revendications précédentes,
dans lequel la première source (2, 25) est conçue pour fournir le premier composant gazeux (air),
dans lequel la seconde source (20) est conçue pour fournir le second composant gazeux (O2),
dans lequel une liaison fluidique peut être établie ou est établie au moins temporairement entre l'élément de liaison d'alimentation (V.1) de l'agencement d'alimentation et la première source (2, 25), et
dans lequel une liaison fluidique (21) peut être établie ou est établie au moins temporairement entre l'entrée de pression amont (V.3) du détendeur (1) et la seconde source (20) (20).

12. Système d'alimentation selon la revendication 11,
**caractérisé en ce que**
la seconde source (20) fournit le second composant gazeux (O2) à une pression plus élevée que la première source (2) fournit le premier composant gazeux (air).

13. Système d'assistance respiratoire (100) pour l'assistance respiratoire artificielle d'un patient (Pt) avec un mélange de gaz comprenant un premier composant gazeux (air) et un second composant gazeux (O2),
dans lequel au moins l'un des deux composants gazeux (air, O2) est de l'oxygène ou contient de l'oxygène,
dans lequel le système d'assistance respiratoire comprend
- une unité de transport de fluide (2),
- une unité de couplage (9) côté patient et
- un agencement d'alimentation selon l'une des revendications 1 à 10 ou un système d'alimentation selon la revendication 11 ou la revendication 12,
dans lequel l'unité de couplage (9) côté patient peut être reliée ou est reliée au moins temporairement à un patient (Pt),
dans lequel une liaison fluidique peut être établie ou est établie au moins temporairement entre l'élément de liaison d'alimentation (V.1) de l'agencement d'alimentation et une première source (2, 25), à savoir une source pour le premier composant gazeux (air),
dans lequel l'unité de guidage de fluide (2) est conçue pour transporter le premier composant gazeux (air) depuis la première source (2, 25) à travers le premier canal (K.1) jusqu'au point de mélange (8),
dans lequel une liaison fluidique (21) est établie ou peut être établie au moins temporairement entre l'entrée de pression amont (V.3) du détendeur (1) et une seconde source (20), à savoir une source pour le second composant gazeux (O2), et
dans lequel le système d'assistance respiratoire (100) est conçu pour
- effectuer des courses d'assistance respiratoire et
- à chaque course d'assistance respiratoire, conduire une quantité du mélange de gaz à travers le canal d'inspiration (K.30) vers l'unité de couplage (9) côté patient.

14. Système d'assistance respiratoire (100) selon la revendication 14, **caractérisé en ce que**
la première soupape (4.1) est conçue pour modifier le débit volumique à travers le premier canal (K.1),
dans lequel l'appareil de commande (3) est conçu pour
- déduire, en fonction d'une courbe de consigne temporelle prédéfinie du débit volumique (Vol') du mélange de gaz, une courbe de consigne du débit volumique du premier composant gazeux (air) et
- commander la première soupape (4.1) avec l'objectif de régulation que le flux volumique effectif à travers le premier canal (K.1) soit égal à la courbe de consigne déduite du flux volumique du premier composant gazeux (air).

15. Système d'assistance respiratoire (100) selon la revendication 13 ou la revendication 14,
**caractérisé en ce que**
le système d'assistance respiratoire (100) comprend une seconde soupape (4.2),
dans lequel la seconde soupape (4.2) est conçue pour modifier le débit volumique à travers le second canal (K.2), et
dans lequel l'appareil de commande (3) est conçu pour
- déduire, en fonction d'une courbe de consigne temporelle prédéfinie du débit volumique (Vol') du mélange de gaz, une courbe de consigne du débit volumique du second composant gazeux (O2) et
- commander la seconde soupape (4.2) avec l'objectif de régulation que le flux volumique effectif à travers le second canal (K.2) soit égal à la courbe de consigne déduite du flux volumique du second composant gazeux (O2).
